# EUROPEAN PATENT SPECIFICATION

(11) **EP 4 132 648 B1**
(45) Date of publication and mention of the grant of the patent: **08.07.2026**
(21) Application number: 21721779.3
(22) Date of filing: 08.04.2021
(51) Int. Cl.: A61K 31/135, A61P 15/00

(54) **CYCLOBENZAPRINE TREATMENT FOR SEXUAL DYSFUNCTION**
CYCLOBENZAPRINBEHANDLUNG FÜR SEXUELLE DYSFUNKTION
TRAITEMENT PAR CYCLOBENZAPRINE POUR TRAITER UNE DYSFONCTION SEXUELLE

(30) Priority: 08.04.2020 US 202063007251 P
(43) Date of publication of application: 15.02.2023
(73) Proprietor: Tonix Pharma Limited, Dublin A96 HW25 (IE)
(72) Inventor: PARMENTER, Megan Elizabeth, Mineola, NY 11501 (US); SULLIVAN, Gregory M., New York, NY 10024 (US)
(74) Representative: HG Law International LLP
(86) International application number: PCT/US2021/026492
(87) International publication number: WO 2021/207561

(56) References cited:
- US-A1- 2020 060 997
- KRAUS MOLLY B ET AL: "Painful Ejaculation with Cyclobenzaprine: A Case Report and Literature Review", SEXUAL MEDICINE,, vol. 3, no. 4, 30 November 2015 (2015-11-30), pages 343 - 345, XP009528347, ISSN: 2050-1161, DOI: 10.1002/SM2.93

## Description

### CROSS-REFERENCE TO RELATED APPLICATIONS

This application claims priority and benefit from United States Provisional Application No. 63/007,251, filed April 8, 2020.

### TECHNICAL FIELD

The present disclosure is directed to pharmaceutical compositions or combinations in accordance with the appended claims.

### BACKGROUND

There is increasing documentation of the comorbidity of sexual dysfunction (SD) and posttraumatic stress disorder (PTSD) among veterans (Letica-Crepulja et al., 2019). Compared to those without any mental health diagnosis or with a mental health diagnosis other than PTSD, veterans with PTSD are at a higher risk of SD. This is true regardless of chronicity of symptoms, age or other health concerns, suggesting that the mechanisms underlying such dysfunction are directly related to PTSD (Hirsch, 2009). Common types of SD in males include erectile dysfunction, sexual disinterest, and premature ejaculation; whereas common types of SD in females include fear of sex, arousal problems, orgasm problems, sexual disinterest, and vaginal pain. Moreover, the relationship between sexual functioning and psychological well-being is stronger for females than males (Rosen & Bauchman, 2008).

Despite the importance of addressing co-morbid symptoms, the topic of SD is often overlooked clinically and underexamined in the research literature (Tran et al., 2015). In terms of treatment, experts recommend that evidence-based treatment for PTSD should be the first priority. However, treating PTSD does not necessarily resolve symptoms of SD. For example, when compared with a no-treatment control group, women who participated in cognitive behavioral therapy for PTSD showed significant reductions in symptoms of PTSD but did not show significant improvements in sexual functioning (Cohen & Hien, 2006). In addition, serotonin reuptake inhibitors (SSRIs) and benzodiazepines, commonly prescribed psychiatric medications for the treatment of PTSD, are shown to exacerbate pre-existing SD, as well as contribute to poor compliance and refusal of medication (Keller, McGarvey, Clayton, 2006).

Cyclobenzaprine and its pharmaceutically acceptable salts is a serotonin-2A, alpha-1-adrenergic, and histamine-1 receptor antagonist that is being developed to target sleep disturbance and hyperarousal in PTSD as a treatment for PTSD. A Phase 2 study of cyclobenzaprine-HCl in military-related PTSD had very low rates of adverse events related to sexual function in both the drug and placebo groups. Therefore, in the subsequent Phase 3 study in military-related PTSD, systematic study was undertaken to assess the effects of treatment on female and male sexual functioning.

US 2020/060997 A1 discloses the treatment of female sexual disorders using trace amine associated receptor (TAAR) agonists. Cyclobenzaprine is mentioned in that document only as one of numerous optional formulation agents, without any disclosure of therapeutic activity for sexual dysfunction.

Kraus et al. (Sexual Medicine, 2015, vol. 3, no. 4, pages 343-345) reports a case of painful ejaculation as an adverse effect of cyclobenzaprine in a male patient. That document does not relate to the therapeutic treatment of female sexual dysfunction.

### SUMMARY OF THE DISCLOSURE

The present disclosure is directed to pharmaceutical compositions or combinations in accordance with the appended claims. It will be appreciated that, while discussed herein for context, methods of treating the human or animal body by therapy are not claimed. For example, methods discussed herein, but not claimed, include:
1. A method for treating or preventing sexual dysfunction and associated symptoms thereof, comprising administering to a female subject in need or at risk thereof, a pharmaceutical composition comprising a therapeutically effective amount of cyclobenzaprine and a pharmaceutically acceptable carrier.
2. The method of embodiment 1, wherein the cyclobenzaprine is a free base or a pharmaceutically acceptable salt thereof.
3. The method of embodiment 1 or 2, wherein the pharmaceutically acceptable salt of cyclobenzaprine is a cyclobenzaprine acid salt.
4. The method of embodiment 3, wherein the cyclobenzaprine acid salt is cyclobenzaprine-HCl.
5. The method of any one of embodiments 1-4, wherein the cyclobenzaprine or pharmaceutically salt thereof is in the form of a eutectic.
6. The method of embodiment 5, wherein the eutectic is a mannitol eutectic.
7. The method of embodiment 6, wherein the mannitol eutectic is selected for the group consisting of a 75% ± 2% cyclobenzaprine-HCl and 25% ± 2% mannitol eutectic, a 65% ± 2% cyclobenzaprine-HCl and 35% ± 2% δ-mannitol eutectic, a mixture of a 75% ± 2% cyclobenzaprine-HCl and 25% ± 2% β-mannitol and a 65% ± 2% cyclobenzaprine-HCl and 35% ± 2% δ-mannitol eutectic, and a granule comprising an outer layer of a 65% ± 2% cyclobenzaprine-HCl and 35% ± 2% δ-mannitol eutectic and an inner layer of β-mannitol.
8. The method of any one of embodiments 1-7, wherein the composition comprising a pharmaceutically acceptable salt of cyclobenzaprine further comprises a basifying agent.
9. The method of embodiment 8, wherein the basifying agent is selected from a group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate, disodium citrate and trisodium citrate.
10. The method according to embodiment 9, wherein the basifying agent is dipotassium hydrogen phosphate.
11. The method of embodiment 1, wherein the composition comprises between 0.1 mg and 30 mg of cyclobenzaprine or a pharmaceutically acceptable salt thereof.
12. The method of embodiment 11, wherein the composition comprises between 1 mg and 20 mg of cyclobenzaprine or a pharmaceutically acceptable salt thereof.
13. The method of embodiment 12, wherein the composition comprises less than 10 mg of cyclobenzaprine or a pharmaceutically acceptable salt thereof.
14. The method of embodiment 12, wherein the composition comprises less than 5 mg of cyclobenzaprine or a pharmaceutically acceptable salt thereof.
15. The method of embodiment 13, wherein the composition comprises about 5.6 mg of cyclobenzaprine or a pharmaceutically acceptable salt thereof.
16. The method of embodiment 15, wherein the composition comprises about 5.6 mg of cyclobenzaprine-HCl.
17. The method of embodiment 13, wherein the composition comprises about 2.8 mg of cyclobenzaprine or a pharmaceutically acceptable salt thereof.
18. The method of embodiment 17, wherein the composition comprises about 2.8 mg of cyclobenzaprine-HCl.
19. The method of embodiment 17 or 18, wherein the composition is administered simultaneously or sequentially in two dosage units, and wherein the combined amount of the composition in the two dosage units is about 5.6 mg of cyclobenzaprine or a pharmaceutically acceptable salt.
20. The method of embodiment 19, wherein the composition is administered simultaneously in two dosage units, and wherein each dosage unit comprises about 2.8 mg of cyclobenzaprine or a pharmaceutically acceptable salt thereof.
21. The method of embodiment 19, wherein the composition is administered sequentially in two dosage units, and wherein each dosage unit comprises about 2.8 mg of cyclobenzaprine or a pharmaceutically acceptable salt thereof.
22. The method of any one of embodiments 1-21, wherein the pharmaceutical composition is administered daily.
23. The method of any one of embodiments 1-22, wherein the composition is administered once daily.
24. The method of embodiment 23, wherein the pharmaceutical composition is formulated for sublingual, buccal, oral, suppository, intravenous, intramuscular, subcutaneous, inhalational, intranasal, thin film, transdermal, parenteral, rectal, or vaginal administration.
25. The method of embodiment 24, wherein the pharmaceutical composition is formulated for sublingual administration.
26. The method of embodiment 25, wherein the pharmaceutical composition is administered sublingually, buccally, orally, in a suppository, intravenously, intramuscularly, subcutaneously, inhalationally, intranasally, in a thin film, transdermally, parenterally, rectally, or vaginally.
27. The method of embodiment 26, wherein the pharmaceutical composition is administered sublingually.
28. The method of any one of embodiments 1-26, wherein the method further comprises administering sequentially or simultaneously one or more therapeutic agents selected from the group consisting an estrogen receptor modulator, a 5-hydroxytryptamine 1A (5-HT_{1A}) receptor agonist, a 5-hydroxytryptamine 2A (5-HT_{2A}) antagonist, a synthetic or gonadal steroid agent, a phosphodiesterase inhibitor, a melanocortin receptor agonist, an alpha-1-adrenergic receptor antagonist, a beta-adrenergic antagonist, an anticonvulsant or a mood stabilizer, a selective serotonin reuptake inhibitor, a serotonin-norepinephrine reuptake inhibitor, an antidepressant, an anti-anxiety agent, an antipsychotic, an antihistamine, a benzodiazepine, a psychoactive agent, a barbiturate, lithium, an antihypertensive agent, an antilipid agent, a hormonal agent, a gonadotropin-releasing hormone (GnRh) agonist, a contraceptive agent, an anticholinergic agent, an amphetamine, a dopaminergic receptor agonist, an anorexic agent, and a narcotic agent.
29. The method of embodiment 28, wherein the estrogen receptor modulator is ospemifene.
30. The method of embodiment 28, wherein the 5-HT_{1A} receptor agonist or the 5-HT_{2A} receptor agonist is flibanserin.
31. The method of embodiment 28, wherein the dopaminergic receptor agonist is apomorphine.
32. The method of embodiment 28, wherein the steroid agent is tibolone, estrogen, or testosterone.
33. The method of embodiment 28, wherein the phosphodiesterase inhibitor is sildenafil or tadalafil.
34. The method of embodiment 28, wherein the melanocortin receptor agonist is bremelanotide.
35. The method of embodiment 28, wherein the alpha-1- adrenergic receptor antagonist is prazosin, terazosin, doxazosin, silodosin, alfuzosin, or tamsulosin.
36. The method of embodiment 28, wherein the beta adrenergic receptor antagonist is propranolol, bucindolol, carteolol, carvedilol, labetalol, nadolol, oxprenolol, penbutolol, pindolol, sotalol, timolol, acebutolol, atenolol, betaxolol, bisoprolol, celiprolol, metoprolol, nebivolol, esmolol, butaxamine, ICI-118,551, SR 59230A, or nebivolol.
37. The method of embodiment 28, wherein the anticonvulsant or mood stabilizer is carbamazepine, divalproex, dextromethorphan, gabapentin, lamotrigine, oxcarbazepine, pregabalin, tiagabine, topimarate, or valproate.
38. The method of embodiment 28, wherein the selective serotonin reuptake inhibitor is citalopram, escitalopram, fluoxetine, fluvoxamine, paroxetine, or sertraline.
39. The method of embodiment 28, wherein the serotonin-norepinephrine reuptake inhibitor is atomoxetine, duloxetine, desvenlafaxine, levomilnacipran, milnacipran, sibutramine, tramadol, or venlafaxine.
40. The method of embodiment 28, wherein the antidepressant is citalopram, fluoxetine, paroxetine, sertraline, escitalopram, trazodone, venlafaxine, bupropion, duloxetine, amitriptyline, venlafaxine, mirtazapine, desvenlafaxine, or nortriptyline.
41. The method of embodiment 28, wherein the anti-anxiety agent is lorazepam, oxazepam, or buspirone.
42. The method of embodiment 28, wherein the antipsychotic agent is quetiapine, trazodone, promazine, aripiprazole, ziprasidone, olanzapine, or risperidone.
43. The method of embodiment 28, wherein the antihistamine is acrivastine, azelastine, bilastine, bromodiphenhydramine, brompheniramine, buclizine, barbinoxamine, cetirizine, chlorodiphenhydramine, chlorpheniramine, clemastine, cyclizine, cyproheptadine, desloratadine, dexbrompheniramine, dexchlorpheniramine, dimenhydrinate dimetindene, diphenhydramine, doxylamine, ebastine, embramine, fexofenadine, hydroxyzine, levocabastine, levocetirizine, loratadine, meclizine, mirtazapine, olopatadine, orphenadrine, phenindamine, pheniramine, phenyltoloxamine, promethazine, quetiapine, rupatadine, tripelennamine, triprolidine, levocetirizine, desloratadine, pyrilamine, cimetidine, famotidine, lafutidine, nizatidine, ranitidine, roxatidine, tiotidine, clobenpropit, ABT-239, ciproxifan, conessine, A-349,821, thioperamide, thioperamide, JNJ 7777120, and VUF-6002.
44. The method of embodiment 28, wherein the benzodiazepine is quazepam, chlordiazepoxide, flurazepam, alprazolam, clorazepate, diazepam, estazolam, clonazepam, oxazepam, triazolam, lorazepam, temazepam, clobazam, and midazolam.
45. The method of embodiment 28, wherein the hormonal agent is oxytocin, estrogen, or testosterone.
46. The method of embodiment 1, wherein the female subject has female genital organs by birth, reconstructive surgery or sex reassignment surgery.
47. The method of embodiment 46, wherein the female subject is premenopausal, perimenopausal, or postmenopausal.
48. The method of any one of embodiments 1-45 and 46-47, wherein the sexual dysfunction is associated with the use of one or more agents selected from a group consisting of an antidepressant, an anxiolytic, an antihypertensive agent, a chemotherapy agent, a hormonal agent, a corticosteroid agent, an antipsychotic, an antihistamine, a benzodiazepine, a psychoactive agent, a barbiturate, lithium, an antihypertensive agent, an antilipid agent, a gonadotropin-releasing hormone (GnRh) agonist, a contraceptive, an anticholinergic agent, an amphetamine, an anorexic agent, and a narcotic agent.
49. The method of any one of embodiments 1-45 and 46-67, wherein the sexual dysfunction is associated with a medical or mental health condition.
50. The method of embodiment 49, wherein the sexual dysfunction is a desire disorder, an arousal disorder, an orgasm disorder, or a sexual pain disorder.
51. The method of embodiment 50, wherein the sexual dysfunction is associated with one or more of the following symptoms: sexual aversion, low sexual desire or interest, fear of sex, difficulty with arousal, inability to become aroused or maintain arousal during sexual activity, persistent or recurrent difficulty in achieving orgasm after sufficient sexual arousal and ongoing stimulation, and pain associated with sexual stimulation or vaginal contact.
52. The method of embodiment 49, wherein the medical condition is selected from a group consisting of a cardiovascular disease, obesity, a cancer, a pulmonary condition, a kidney condition, a bladder condition, a rectal condition, a bowel condition, a hepatic condition, a gynecological condition, an autoimmune disorder, a hormonal condition, a viral infection, bacterial infection, a parasitic infection, and a prion infection.
53. The method of embodiment 52, wherein the cardiovascular disease is selected from the group consisting of heart disease, hypertension and peripheral vascular disease.
54. The method of embodiment 52, wherein the cancer is selected from the group consisting of breast cancer, ovarian cancer, cervical cancer, endometrial cancer, gestational trophoblastic disease, uterine sarcoma, vaginal cancer, vulvar cancer, pancreatic cancer, rectal cancer, renal cell cancer, skin cancer, brain cancer, head and neck cancer, lung cancer, thyroid cancer, bladder cancer, esophageal cancer, mesothelioma, glioblastoma, thymic carcinoma, lymphoma, leukemia, myeloma, hematologic malignancy, and colon or gastrointestinal cancer.
55. The method of embodiment 52, wherein the pulmonary condition is selected from the group consisting of pneumonia, tuberculosis, emphysema, pulmonary edema, acute respiratory distress syndrome, pneumoconiosis, pulmonary embolism, pulmonary hypertension, pleural effusion, pneumothorax, and mesothelioma.
56. The method of embodiment 52, wherein the autoimmune disease is selected from the group consisting of multiple sclerosis, type I diabetes, rheumatoid arthritis, psoriasis, systemic lupus erythematosus, Addison's disease, Graves disease, Sjögren's syndrome, Myasthenia gravis, pernicious anemia, and celiac disease.
57. The method of embodiment 52, wherein the hepatic condition is selected from the group consisting of hepatitis, fatty liver disease, liver cancer, hemochromatosis, and Wilson disease.
58. The method of embodiment 52, wherein the gynecological condition is selected from the group consisting of menopause, peritonitis, uterine retrogression, fibroids, endometritis, uterine cysts, cystocele, rectocele, uterine prolapse, hysterectomy, oophorectomy, salpingectomy, and hormone fluctuation.
59. The method of embodiment 52, wherein the bladder condition is selected from the group consisting of urethritis, interstitial cystitis, and urinary tract infection.
60. The method of embodiment 52, wherein the kidney condition is selected from the group consisting of Chronic Kidney Disease (CKD), diabetes, anorexia nervosa, high blood pressure, high cholesterol, lupus, multiple myeloma and hemolytic uremic syndrome.
61. The method of embodiment 52, wherein the hormonal condition is associated with menopause, perimenopause, pregnancy, or childbirth.
62. The method of embodiment 52, wherein the viral infection is caused by human papilloma virus hepatitis C virus, or herpes simplex virus.
63. The method of embodiment 52, wherein the bacterial infection is caused by ardnerella vaginalis.
64. The method of embodiment 49, wherein the mental health condition is one or more conditions selected from a group consisting of psychological conditions, mood disorder, trauma and stressor related disorder, neurodegenerative disorder, and anxiety disorder.
65. The method of embodiment 64, wherein the psychologic condition is sexual, emotional, or physical trauma or abuse.
66. The method of embodiment 64, wherein the mood disorder is a depressive disorder, a bipolar disorder, or a substance-induced disorder.
67. The method of embodiment 64, wherein the trauma and stressor related disorder is a post-traumatic stress disorder, acute stress disorder, adjustment disorder, or reactive attachment disorder.
68. The method of embodiment 64, wherein the neurodegenerative disorder is Mild Cognitive Impairment, amnestic Mild Cognitive Impairment, Parkinson's disease, Huntington's disease, Alzheimer's Disease, dementia, Amyotrophic lateral sclerosis, or motor neuron disease.
69. The method of embodiment 64, wherein the anxiety disorder is panic, generalized anxiety disorder, specific phobia, or social phobia.

Some embodiments of this disclosure are:
1. A combination comprising a therapeutically effective amount of cyclobenzaprine or a pharmaceutically acceptable salt thereof and optionally one or more therapeutic agents selected from the group consisting of an estrogen receptor modulator, a 5-hydroxytryptamine 1A (5-HT_{1A}) receptor agonist, a steroid agent, a phosphodiesterase inhibitor, a melanocortin receptor agonist, an alpha-1- adrenergic receptor antagonist, a beta-adrenergic antagonist, an anticonvulsant or a mood stabilizer, a selective serotonin reuptake inhibitor, a serotonin-norepinephrine reuptake inhibitor, an antidepressant, an anti-anxiety agent, an antipsychotic, an antihistamine, a benzodiazepine, a psychoactive agent, a barbiturate, lithium, an antihypertensive agent, an antilipid agent, a hormonal agent, a gonadotropin-releasing hormone (GnRh) agonist, a contraceptive, an anticholinergic, an amphetamine, an anorexic agent, and a narcotic agent.
2. The combination of embodiment 1, wherein the cyclobenzaprine or salt thereof and the one or more agents are in the same dosage form or in separate dosage forms packaged together or packaged separately, wherein the cyclobenzaprine or salt thereof and the one or more therapeutic agents are administered simultaneously or sequentially.
3. The combination of embodiment 1, wherein the one or more therapeutic agents is ospemifene, flibanserin, tibolone, estrogen, or testosterone, sildenafil, tadalafil, bremelanotide, prazosin, terazosin, doxazosin, silodosin, alfuzosin, tamsulosin, propranolol, bucindolol, carteolol, carvedilol, labetalol, nadolol, oxprenolol, penbutolol, pindolol, sotalol, timolol, acebutolol, atenolol, betaxolol, bisoprolol, celiprolol, metoprolol, nebivolol, esmolol, butaxamine, ICI-118,551, SR 59230A, nebivolol, carbamazepine, divalproex, dextromethorphan, gabapentin, lamotrigine, oxcarbazepine, pregabalin, tiagabine, topimarate, valproate, citalopram, escitalopram, fluoxetine, fluvoxamine, paroxetine, sertraline, atomoxetine, duloxetine, desvenlafaxine, levomilnacipran, milnacipran, sibutramine, tramadol, venlafaxine, citalopram, fluoxetine, paroxetine, sertraline, escitalopram, trazodone, venlafaxine, bupropion, duloxetine, amitriptyline, venlafaxine, mirtazapine, desvenlafaxine, nortriptyline, lorazepam, oxazepam, buspirone, quetiapine, trazodone, promazine, aripiprazole, ziprasidone, olanzapine, risperidone, acrivastine, azelastine, bilastine, bromodiphenhydramine, brompheniramine, buclizine, barbinoxamine, cetirizine, chlorodiphenhydramine, chlorpheniramine, clemastine, cyclizine, cyproheptadine, desloratadine, dexbrompheniramine, dexchlorpheniramine, dimenhydrinate dimetindene, diphenhydramine, doxylamine, ebastine, embramine, fexofenadine, hydroxyzine, levocabastine, levocetirizine, loratadine, meclizine, mirtazapine, olopatadine, orphenadrine, phenindamine, pheniramine, phenyltoloxamine, promethazine, quetiapine, rupatadine, tripelennamine, triprolidine, levocetirizine, desloratadine, pyrilamine, cimetidine, famotidine, lafutidine, nizatidine, ranitidine, roxatidine, tiotidine, clobenpropit, ABT-239, ciproxifan, conessine, A-349,821, thioperamide, thioperamide, JNJ 7777120, VUF-6002, quazepam, chlordiazepoxide, flurazepam, alprazolam, clorazepate, diazepam, estazolam, clonazepam, oxazepam, triazolam, lorazepam, temazepam, clobazam, midazolam, or oxytocin.
4. The combination of embodiment 1, wherein the cyclobenzaprine is a free base or a pharmaceutically acceptable salt thereof.
5. The combination of any one of embodiments 2-4, wherein the pharmaceutically acceptable salt of cyclobenzaprine is a cyclobenzaprine acid salt.
6. The combination of embodiment 5, wherein the cyclobenzaprine acid salt is cyclobenzaprine-HCl.
7. The combination of any one of embodiments 2-6, wherein the cyclobenzaprine or pharmaceutically salt thereof is in the form of a eutectic.
8. The combination of embodiment 7, wherein the eutectic is a mannitol eutectic.
9. The combination of embodiment 8, wherein the eutectic is selected for the group consisting of a 75% ± 2% cyclobenzaprine-HCl and 25% ± 2% mannitol eutectic, a 65% ± 2% cyclobenzaprine-HCl and 35% ± 2% δ-mannitol eutectic, a mixture of a 75% ± 2% cyclobenzaprine-HCl and 25% ± 2% β-mannitol and a 65% ± 2% cyclobenzaprine-HCl and 35% ± 2% δ-mannitol eutectic, and a granule comprising an outer layer of a 65% ± 2% cyclobenzaprine-HCl and 35% ± 2% δ-mannitol eutectic and an inner layer of β-mannitol.
10. The combination of any one of embodiments 2-9, wherein the combination comprises a pharmaceutically acceptable salt of cyclobenzaprine and a basifying agent.
11. The combination of embodiment 10, wherein the basifying agent is selected from a group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate, disodium citrate and trisodium citrate.
12. The combination according to embodiment 11, wherein the basifying agent is dipotassium hydrogen phosphate.
13. The combination of embodiment 12, wherein the combination comprises between 0.1 mg and 30 mg of cyclobenzaprine or a pharmaceutically acceptable salt thereof.
14. The combination of embodiment 13, where the combination comprises between 1 mg and 20 mg of cyclobenzaprine or a pharmaceutically acceptable salt thereof.
15. The combination of embodiment 14, wherein the combination comprises less than 10 mg of cyclobenzaprine or a pharmaceutically acceptable salt thereof.
16. The combination of embodiment 14, wherein the combination comprises less than 5 mg of cyclobenzaprine or a pharmaceutically acceptable salt thereof.
17. The combination of embodiment 15, wherein the combination comprises about 5.6 mg of cyclobenzaprine or a pharmaceutically acceptable salt thereof.
18. The combination of embodiment 17, wherein the composition comprises about 5.6 mg of cyclobenzaprine-HCl.
19. The combination of embodiment 15, wherein the combination comprises about 2.8 mg of cyclobenzaprine or a pharmaceutically acceptable salt thereof.
20. The combination of embodiment 19, wherein the combination comprises about 2.8 mg of cyclobenzaprine.

### DETAILED DESCRIPTION

The present disclosure discusses methods and claims pharmaceutical compositions for treating sexual dysfunction and associated symptoms in a subject in need or at risk thereof, wherein the pharmaceutical compositions comprise a therapeutically effective amount of cyclobenzaprine and a pharmaceutically acceptable carrier, optionally in combination with one or more therapeutic or non-therapeutic agents.

### Definitions

The term "herein" means the entire application.

Unless otherwise defined herein, scientific and technical terms used in this application shall have the meanings that are commonly understood by those of ordinary skill in the art. In case of conflict, the present specification, including definitions, will control.

It should be understood that any of the embodiments described herein, including those described under different aspects of the disclosure and different parts of the specification can be combined with one or more other embodiments of this disclosure, unless explicitly disclaimed or improper. Combination of embodiments are not limited to those specific combinations claimed via the multiple dependent claims.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The term "including," as used herein, means "including but not limited to." "Including" and "including but not limited to" are used interchangeably. Thus, these terms will be understood to imply the inclusion of a stated integer (or components) or group of integers (or components), but not the exclusion of any other integer (or components) or group of integers (or components).

As used herein, the term "about" refers to a value or parameter that includes (and describes) embodiments that are directed to that value or parameter per se. For example, description referring to "about X" includes description of "X". As used herein, the term "about" permits a variation of ±10% within the range of the significant digit. Numeric ranges are inclusive of the numbers defining the range.

Any example(s) following the term "e.g." or "for example" is not meant to be exhaustive or limiting.

Unless otherwise required by context, singular terms shall include pluralities and plural terms shall include the singular.

The articles "a", "an" and "the" are used herein to refer to one or to more than one (i.e., to at least one) of the grammatical object of the article.

Where aspects or embodiments are described in terms of a Markush group or other grouping of alternatives, the present application encompasses not only the entire group listed as a whole, but each member of the group individually and all possible subgroups of the main group, and also the main group absent one or more of the group members.

Exemplary methods and materials are described herein, although methods and materials similar or equivalent to those described herein can also be used in the practice or testing of the various aspects and embodiments of this disclosure. The materials, methods, and examples are illustrative only and not intended to be limiting.

In order that the disclosure may be more readily understood, certain terms are first defined. These definitions should be read in light of the remainder of the disclosure as understood by a person of ordinary skill in the art. Unless defined otherwise, all technical and scientific terms used herein have the same meaning as commonly understood by a person of ordinary skill in the art. Additional definitions are set forth throughout the detailed description.

As used herein, the term "treat" and its cognates refer to a full or partial amelioration, improvement, or modulation of sexual dysfunction or at least one discernible symptom therein. In some embodiments, "treat at least one discernible symptom" refers to an improvement of desire and/or interest. In some embodiments, "treat" refers to an improvement of pleasure. In some embodiments, "treat of at least one discernible symptom" refers to improvement in desire and/or frequency. In some embodiments, "treat" refers to an improvement in arousal and/or excitement. In some embodiments, "treat at least one discernible symptom" refers to improvement in orgasm and/or completion. In some embodiments, "treat at least one discernible symptom" refers to improvement in a desire disorder. In some embodiments, "treat at least one discernible symptom" refers to improvement in an arousal disorder. In some embodiments, "treat at least one discernible symptom" refers to improvement in an orgasm disorder. In some embodiments, "treat at least one discernible symptom" refers to improvement in a sexual pain disorder.

In some embodiments of this disclosure, the cyclobenzaprine is an acid salt of cyclobenzaprine. In other embodiments, the acid salt is cyclobenzaprine-HCl.

In other embodiments, the acid salt is combined with a basifying agent. In some embodiments, the basifying agent is an ingredient (and excipient) in a tablet or other formulation, and the basifying agent exerts its effects during the time the formulation is being dispersed in the mucous material, including buccal and sublingual tissue, while parts of the formulation are dissolving in the mucous material and for a period of time after the tablet is dissolved in the mucous material.

The " basifying agent" included in some embodiments of this disclosure is selected from a group consisting of potassium dihydrogen phosphate (monopotassium phosphate, monobasic potassium phosphate, KH₂PO₄), dipotassium hydrogen phosphate (dipotassium phosphate, dibasic potassium phosphate, K₂HPO₄), tripotassium phosphate (K₃PO₄), sodium dihydrogen phosphate (monosodium phosphate, monobasic sodium phosphate, NaH₂PO₄), disodium hydrogen phosphate (disodium phosphate, dibasic sodium phosphate, Na₂HPO₄), trisodium phosphate (Na₃PO₄), bicarbonate or carbonate salts, dipotassium citrate, tripotassium citrate, disodium citrate, trisodium citrate, borate, hydroxide, silicate, nitrate, dissolved ammonia, the conjugate bases of some organic acids (including bicarbonate), and sulfide. In some embodiments, the basifying agent is dipotassium hydrogen phosphate (K₂HPO₄), potassium dihydrogen phosphate (KH₂PO₄), disodium hydrogen phosphate (Na₂HPO₄), tripotassium citrate or trisodium citrate. A basifying agent that is particularly useful in combination with cyclobenzaprine-HCl is dipotassium hydrogen phosphate (K₂HPO₄). Another basifying agent that is particularly useful in combination with cyclobenzaprine-HCl is potassium dihydrogen phosphate (KH₂PO₄). Another basifying agent that is particularly useful in combination with cyclobenzaprine-HCl is disodium hydrogen phosphate (Na₂HPO₄). Another basifying agent that is particularly useful in combination with cyclobenzaprine-HCl is tripotassium citrate. Another basifying agent that is particularly useful in combination with cyclobenzaprine-HCl is trisodium citrate.

In some embodiments of this disclosure, the cyclobenzaprine or its acid salt is present in a eutectic. In some embodiments, the eutectic includes mannitol. In some aspects, the mannitol is beta mannitol. In other embodiments, the mannitol is delta mannitol. In some aspects, the eutectic is a eutectic of the cyclobenzaprine-HCl and mannitol is selected from the group consisting of a 75% ± 2% cyclobenzaprine-HCl and 25% ± 2% mannitol eutectic, a 65% ± 2% cyclobenzaprine-HCl and 35% ± 2% δ-mannitol eutectic, a mixture of a 75% ± 2% cyclobenzaprine-HCl and 25% ± 2% β-mannitol and a 65% ± 2% cyclobenzaprine-HCl and 35% ± 2% δ-mannitol eutectic, and a granule comprising an outer layer of a 65% ± 2% cyclobenzaprine-HCl and 35% ± 2% δ-mannitol eutectic and an inner layer of β-mannitol. See, e.g., WO2014/145156 and WO2016/044796. It should be understood that the "cyclobenzaprine-HCl" eutectic of this disclosure refers to any of these eutectics or granules. In some aspects, the eutectic is combined with a basifying agent. See, e.g., WO2013/188847.

As used herein, the term a "eutectic" or "in the form of a eutectic" refers to a mixture of chemical compounds or elements that has a single chemical composition that melts at a lower temperature than any other composition made up of the same ingredients. A composition comprising a eutectic is known as the eutectic composition and its melting temperature is known as the eutectic temperature. Eutectic compositions often have a higher stability and/or dissolution rates than their non-eutectic counterparts. Because eutectics enhance dissolution, they can be employed to increase permeability in solid dispersions and dispersion systems.

### Method for treating

In some embodiments, the present disclosure provides a method for treating, improving and/or preventing sexual dysfunction and associate symptoms thereof, comprising administering to a female subject in need or at risk thereof, a pharmaceutical composition comprising a therapeutically effective amount of cyclobenzaprine and a pharmaceutically acceptable carrier.

In some embodiments, the method for treating improving and/or preventing sexual dysfunction comprises administering a pharmaceutical composition comprising a pharmaceutically acceptable acid salt of cyclobenzaprine and a basifying agent. In some embodiments, the pharmaceutical composition comprises a eutectic of a pharmaceutically acceptable salt of cyclobenzaprine and mannitol, which optionally is combined with a basifying agent. The composition of this disclosure may be administered in one, two or more daily doses. In some embodiments, the method for treating and/or preventing sexual dysfunction comprises administering a daily dose between 0.1 mg and 30 mg of cyclobenzaprine or a pharmaceutically acceptable salt thereof. In some embodiments, the daily dose is between 1 mg and 20 mg of cyclobenzaprine or a pharmaceutically acceptable sale thereof. In some embodiments, the daily dose is less than 10 mg of cyclobenzaprine or a pharmaceutically acceptable salt thereof. In some embodiments, the daily dose is less than 5 mg of cyclobenzaprine or a pharmaceutically acceptable salt thereof. In some embodiments, the daily dose comprises about 5.6 mg of cyclobenzaprine or a pharmaceutically acceptable salt thereof. In some embodiments, the daily dose comprises about 5.6 mg of cyclobenzaprine-HCl. In some embodiments, the daily dose comprises about 2.8 mg of cyclobenzaprine or a pharmaceutically acceptable salt thereof. In some embodiments, the daily dose comprises about 2.8 mg of cyclobenzaprine-HCl. In some embodiments, the method for treating and/or preventing sexual dysfunction comprises administering simultaneously or sequentially two dosage units of cyclobenzaprine, and wherein each dosage unit comprises about 2.8 mg of cyclobenzaprine or a pharmaceutically acceptable salt thereof. In some embodiments, each of the two dosage units comprises about 2.8 mg of cyclobenzaprine-HCl. In some embodiments, the method for treating and/or preventing sexual dysfunction comprises administering a therapeutically effective amount of cyclobenzaprine or a pharmaceutically acceptable salt thereof daily. In some embodiments, the method for treating and/or preventing sexual dysfunction comprises administering a therapeutically effective amount of cyclobenzaprine or a pharmaceutically acceptable salt thereof once daily.

In some embodiments, the method for treating and/or preventing sexual dysfunction comprises administering a pharmaceutical composition comprising a therapeutically effective amount of cyclobenzaprine or a pharmaceutically acceptable salt thereof, wherein the pharmaceutical composition is formulated for sublingual, buccal, oral, suppository, intravenous, intramuscular, subcutaneous, inhalational, intranasal, thin film, transdermal, parenteral, rectal, or vaginal administration. In some embodiments, the pharmaceutical composition is formulated for sublingual administration. In some embodiments, the pharmaceutical composition is formulated for buccal administration. In some embodiments, the pharmaceutical composition is formulated for oral administration. In some embodiments, the pharmaceutical composition is formulated for suppository administration. In some embodiments, the pharmaceutical composition is formulated for intravenous administration. In some embodiments the pharmaceutical composition is formulated for intramuscular administration. In some embodiments, the pharmaceutical composition is formulated for subcutaneous administration. In some embodiments, the pharmaceutical composition is formulated for intranasal administration. In some embodiments, the pharmaceutical composition is formulated for transdermal administration. In some embodiments, the pharmaceutical composition is formulated for parenteral administration. In some embodiments, the pharmaceutical composition is formulated for rectal administration. In some embodiments, the pharmaceutical composition is formulated for vaginal administration.

In some embodiments, the methods of this disclosure further comprises administering sequentially or simultaneously, with a composition of this disclosure comprising a cyclobenzaprine or pharmaceutically acceptable salt thereof, one or more therapeutic agents selected from the group consisting an estrogen receptor modulator, a 5-hydroxytryptamine 1A (5-HT_{1A}) receptor agonist, a 5-hydroxytryptamine 2A (5-HT_{2A}) antagonist, a synthetic or gonadal steroid agent, a phosphodiesterase inhibitor, a melanocortin receptor agonist, an alpha-1-adrenergic receptor antagonist, a beta-adrenergic antagonist, an anticonvulsant or a mood stabilizer, a selective serotonin reuptake inhibitor, a serotonin-norepinephrine reuptake inhibitor, an antidepressant, an anti-anxiety agent, an antipsychotic, an antihistamine, a benzodiazepine, a psychoactive agent, a barbiturate, lithium, an antihypertensive agent, an antilipid agent, a hormonal agent, a gonadotropin-releasing hormone (GnRh) agonist, a contraceptive agent, an anticholinergic agent, an amphetamine, a dopaminergic receptor agonist, an anorexic agent, and a narcotic agent. In some embodiments, the one or more therapeutic agents are administered sequentially or simultaneously with a composition of this disclosure comprising cyclobenzaprine-HCl.

In some embodiments, the sexual dysfunction or risk thereof is in a female subject. In some embodiments, the subject has female genital organs by birth, reconstructive surgery or sex reassignment surgery. In some embodiments, the female subject is premenopausal, perimenopausal, or postmenopausal.

In some embodiments, the sexual dysfunction may be associated with the use of one or more agents selected from a group consisting of an antidepressant, an anxiolytic, an antihypertensive agent, a chemotherapy agent, a hormonal agent, a corticosteroid agent, an antipsychotic, an antihistamine, a benzodiazepine, a psychoactive agent, a barbiturate, lithium, an antihypertensive agent, an antilipid agent, a gonadotropin-releasing hormone (GnRh) agonist, a contraceptive, an anticholinergic agent, an amphetamine, an anorexic agent, and a narcotic agent.

In some embodiments, the sexual dysfunction is associated with a medical or mental health condition. In some embodiments, the sexual dysfunction is associated with a medical condition, wherein the medical condition is selected from a group consisting of a cardiovascular disease, obesity, a cancer, a pulmonary condition, a kidney condition, a bladder condition, a rectal condition, a bowel condition, a hepatic condition, a gynecological condition, an autoimmune disorder, a hormonal condition, a viral infection, bacterial infection, a parasitic infection, and a prion infection. In some embodiments, cardiovascular disease is selected from the group consisting of heart disease, hypertension and peripheral vascular disease. In some embodiments, the cancer is selected from the group consisting of breast cancer, ovarian cancer, cervical cancer, endometrial cancer, gestational trophoblastic disease, uterine sarcoma, vaginal cancer, vulvar cancer, pancreatic cancer, rectal cancer, renal cell cancer, skin cancer, brain cancer, head and neck cancer, lung cancer, thyroid cancer, bladder cancer, esophageal cancer, mesothelioma, glioblastoma, thymic carcinoma, lymphoma, leukemia, myeloma, hematologic malignancy, and colon or gastrointestinal cancer. In some embodiments, the pulmonary condition is selected from the group consisting of pneumonia, tuberculosis, emphysema, pulmonary edema, acute respiratory distress syndrome, pneumoconiosis, pulmonary embolism, pulmonary hypertension, pleural effusion, pneumothorax, and mesothelioma. In some embodiments, the autoimmune disease is selected from the group consisting of multiple sclerosis, type I diabetes, rheumatoid arthritis, psoriasis, systemic lupus erythematosus, Addison's disease, Graves' disease, Sjögren's syndrome, Myasthenia gravis, pernicious anemia, and celiac disease. In some embodiments, the hepatic condition is selected from the group consisting of hepatitis, fatty liver disease, liver cancer, hemochromatosis, and Wilson disease. In some embodiments, the gynecological condition is selected from the group consisting of menopause, peritonitis, uterine retrogression, fibroids, endometritis, uterine cysts, cystocele, rectocele, uterine prolapse, hysterectomy, oophorectomy, salpingectomy, and hormone fluctuation. In some embodiments, the bladder condition is selected from the group consisting of urethritis, interstitial cystitis, and urinary tract infection. In some embodiments, the kidney condition is selected from the group consisting of Chronic Kidney Disease (CKD), diabetes, anorexia nervosa, high blood pressure, high cholesterol, lupus, multiple myeloma and hemolytic uremic syndrome. In some embodiments, the hormonal condition is associated with menopause, perimenopause, pregnancy, or childbirth. In some embodiments, the viral infection is caused by human papilloma virus hepatitis C virus, or herpes simplex virus. In some embodiments, the bacterial infection is caused by Gardnerella vaginalis.

In some embodiments, mental health condition is one or more conditions selected from a group consisting of a psychological condition, a mood disorder, a trauma and a stressor related disorder, a neurodegenerative disorder, and an anxiety disorder. In some embodiments, the psychologic condition is sexual, emotional, or physical trauma or abuse. In some embodiments, the mood disorder is a depressive disorder, a bipolar disorder, or a substance-induced disorder. In some embodiments, the trauma and stressor related disorder is a post-traumatic stress disorder, acute stress disorder, adjustment disorder, or reactive attachment disorder. In some embodiments, the neurodegenerative disorder is Mild Cognitive Impairment, amnestic Mild Cognitive Impairment, Parkinson's disease, Huntington's disease, Alzheimer's Disease, dementia, Amyotrophic lateral sclerosis, or motor neuron disease. In some embodiments, the anxiety disorder is panic, generalized anxiety disorder, specific phobia, or social phobia.

In some embodiments, the sexual dysfunction is a desire disorder, an arousal disorder, an orgasm disorder, or a sexual pain disorder.

In some embodiments, the sexual dysfunction is associated with one or more of the following symptoms: sexual aversion, low sexual desire or interest, fear of sex, difficulty with arousal, inability to become aroused or maintain arousal during sexual activity, persistent or recurrent difficulty in achieving orgasm after sufficient sexual arousal and ongoing stimulation, and pain associated with sexual stimulation or vaginal contact.

### Changes in Sexual Functioning Questionnaire short form (CSFQ-14)

The Changes in Sexual Functioning Questionnaire (CSFQ) is a 36-item clinical and research instrument identifying five scales of sexual functioning. The CSFQ has been shortened to a factor structure of a 14-item version (CSFQ-14), which yields scores for three scales corresponding to the phases of the sexual response cycle (e.g., desire, arousal, and orgasm) as well as the five scales of the original CSFQ (e.g., desire/frequency, desire/interest, arousal/excitement, orgasm/complete, and pleasure). Factor analysis confirms the construct validity of the CSFQ-14 as a global measure of sexual dysfunction.

### DSM-5 Diagnostic Criteria for PTSD and CAPS-5

The Diagnostic and Statistical Manual of Mental Disorders, Fifth Edition (DSM-5) is a diagnostic tool published by the American Psychiatric Association. DSM-5 contains descriptions, symptoms and criteria for diagnosing mental disorders. It also contains common language for clinicians to communicate about their patients to establish consistent and reliable diagnoses that can be used in the research of mental disorders. DSM-5 further provides researchers common language to study the criteria for potential future revisions and to aid the development of medications and other interventions.

The Clinician-Administered PTSD Scale (CAPS) is a semi-structured diagnostic interview that assesses essential features of PTSD as defined by the DSM-5 Diagnostic Criteria for PTSD (Weathers et al., 2017). It can also be used to assess associated features of the diagnostic syndrome (e.g., survivor guilt). The interview is designed to accommodate different time spans post-trauma as the reference point for diagnosis. The CAPS affords the clinician flexibility to inquire about symptoms and diagnostic status over the past week, most recent month, and/or for lifetime diagnosis. Any one, or all three, of the time frames may be used depending on the nature task at hand. Other diagnostic scales or tool based on the DSM-5 Diagnostic Criteria for diagnosing PTSD are also well-known. They include, for example, a PTSD checklist for DSM-5 (PCL-5), a clinician-completed symptom severity, intensity and/or frequency rating scale, and a patient-completed symptom severity, intensity and/or intensity rating scale.

In some embodiments, assessing changes in one or more of the DSM-5 Diagnostic Criteria items for PTSD is based on one or more of a Clinician-Administered PTSD Scale for DSM-5 (CAPS-5), a PTSD checklist for DSM-5 (PCL-5), a clinician-completed symptom severity, intensity, and/or frequency rating scale, or a patient-completed symptom severity, intensity, and/or frequency rating scale. The Clinician-Administered PTSD Scale for DSM-5 (CAPS-5) is a 30-item questionnaire corresponding to the DSM-5 diagnosis for PTSD. CAPS-5 requires the identification of a single index trauma to serve as the basis of symptom inquiry. CAPS-5 asks questions relevant to assessing the dissociative subtype of PTSD (depersonalization and derealization), but no longer includes other associated symptoms (e.g., gaps in awareness). CAPS-5 symptom severity ratings are based on symptom frequency and intensity. However, CAPS-5 items are rated with a single severity score in contrast to previous versions of the CAPS which required separate frequency and intensity scores.

### Pharmaceutical composition

In some embodiments, the present disclosure provides a combination comprising a therapeutically effective amount of cyclobenzaprine or a pharmaceutically acceptable salt thereof of this disclosure and optionally one or more therapeutic agents selected from the group consisting of an estrogen receptor modulator, a 5-hydroxytryptamine 1A (5-HT_{1A}) receptor agonist, a steroid agent, a phosphodiesterase inhibitor, a melanocortin receptor agonist, an alpha-1- adrenergic receptor antagonist, a beta-adrenergic antagonist, an anticonvulsant or a mood stabilizer, a selective serotonin reuptake inhibitor, a serotonin-norepinephrine reuptake inhibitor, an antidepressant, an anti-anxiety agent, an antipsychotic, an antihistamine, a benzodiazepine, a psychoactive agent, a barbiturate, lithium, an antihypertensive agent, an antilipid agent, a hormonal agent, a gonadotropin-releasing hormone (GnRh) agonist, a contraceptive, an anticholinergic, an amphetamine, an anorexic agent, and a narcotic agent. In some embodiments, the estrogen receptor modulator is ospemifene. In some embodiments, the 5-HT_{1A} receptor agonist or the 5-HT_{2A} receptor agonist is flibanserin. In some embodiments, the dopaminergic receptor agonist is apomorphine. In some embodiments, the steroid agent is tibolone, estrogen, or testosterone. In some embodiments, the phosphodiesterase inhibitor is sildenafil or tadalafil. In some embodiments, the melanocortin receptor agonist is bremelanotide. In some embodiments, the alpha-1- adrenergic receptor antagonist is prazosin, terazosin, doxazosin, silodosin, alfuzosin, or tamsulosin. In some embodiments, the beta adrenergic receptor antagonist is propranolol, bucindolol, carteolol, carvedilol, labetalol, nadolol, oxprenolol, penbutolol, pindolol, sotalol, timolol, acebutolol, atenolol, betaxolol, bisoprolol, celiprolol, metoprolol, nebivolol, esmolol, butaxamine, ICI-118,551, SR 59230A, or nebivolol. In some embodiments, the anticonvulsant or mood stabilizer is carbamazepine, divalproex, dextromethorphan, gabapentin, lamotrigine, oxcarbazepine, pregabalin, tiagabine, topimarate, or valproate. In some embodiments, the selective serotonin reuptake inhibitor is citalopram, escitalopram, fluoxetine, fluvoxamine, paroxetine, or sertraline. In some embodiments, the serotonin-norepinephrine reuptake inhibitor is atomoxetine, duloxetine, desvenlafaxine, levomilnacipran, milnacipran, sibutramine, tramadol, or venlafaxine. In some embodiments, the antidepressant is citalopram, fluoxetine, paroxetine, sertraline, escitalopram, trazodone, venlafaxine, bupropion, duloxetine, amitriptyline, venlafaxine, mirtazapine, desvenlafaxine, or nortriptyline. In some embodiments, the anti-anxiety agent is lorazepam, oxazepam, or buspirone. In some embodiments, the antipsychotic agent is quetiapine, trazodone, promazine, aripiprazole, ziprasidone, olanzapine, or risperidone. In some embodiments, the antihistamine is acrivastine, azelastine, bilastine, bromodiphenhydramine, brompheniramine, buclizine, barbinoxamine, cetirizine, chlorodiphenhydramine, chlorpheniramine, clemastine, cyclizine, cyproheptadine, desloratadine, dexbrompheniramine, dexchlorpheniramine, dimenhydrinate dimetindene, diphenhydramine, doxylamine, ebastine, embramine, fexofenadine, hydroxyzine, levocabastine, levocetirizine, loratadine, meclizine, mirtazapine, olopatadine, orphenadrine, phenindamine, pheniramine, phenyltoloxamine, promethazine, quetiapine, rupatadine, tripelennamine, triprolidine, levocetirizine, desloratadine, pyrilamine, cimetidine, famotidine, lafutidine, nizatidine, ranitidine, roxatidine, tiotidine, clobenpropit, ABT-239, ciproxifan, conessine, A-349,821, thioperamide, thioperamide, JNJ 7777120, and VUF-6002. In some embodiments, the benzodiazepine is quazepam, chlordiazepoxide, flurazepam, alprazolam, clorazepate, diazepam, estazolam, clonazepam, oxazepam, triazolam, lorazepam, temazepam, clobazam, and midazolam. In some embodiments, the hormonal agent is oxytocin, estrogen, or testosterone.

In some embodiments, the cyclobenzaprine or a pharmaceutically acceptable salt thereof of this disclosure and the one or more optional therapeutic agents are in the same dosage form or in separate dosage forms packaged together or packaged separately, wherein the cyclobenzaprine or a pharmaceutically acceptable salt thereof and the optional one or more therapeutic agents are administered simultaneously or sequentially. In some embodiments, the cyclobenzaprine or salt and the one or more optional therapeutic agents are in the same dosage form. In some embodiments, the cyclobenzaprine and the one or more optional therapeutic agents are in the separate dosage form. In some embodiments, the cyclobenzaprine or salt and the one or more optional therapeutic agents are packaged together. In some embodiments, the cyclobenzaprine or salt and the one or more optional therapeutic agents are packaged separately. In some embodiments, the cyclobenzaprine or salt and the one or more optional therapeutic agents are administered simultaneously. In some embodiments, the cyclobenzaprine or salt and the one or more optional therapeutic agents are administered sequentially.

In some embodiments, the one or more optional therapeutic agents is ospemifene, flibanserin, tibolone, estrogen, or testosterone, sildenafil, bremelanotide, prazosin, terazosin, doxazosin, silodosin, alfuzosin, tamsulosin, propranolol, bucindolol, carteolol, carvedilol, labetalol, nadolol, oxprenolol, penbutolol, pindolol, sotalol, timolol, acebutolol, atenolol, betaxolol, bisoprolol, celiprolol, metoprolol, nebivolol, esmolol, butaxamine, ICI-118,551, SR 59230A, nebivolol, carbamazepine, divalproex, dextromethorphan, gabapentin, lamotrigine, oxcarbazepine, pregabalin, tiagabine, topimarate, valproate, citalopram, escitalopram, fluoxetine, fluvoxamine, paroxetine, sertraline, atomoxetine, duloxetine, desvenlafaxine, levomilnacipran, milnacipran, sibutramine, tramadol, venlafaxine, citalopram, fluoxetine, paroxetine, sertraline, escitalopram, trazodone, venlafaxine, bupropion, duloxetine, amitriptyline, venlafaxine, mirtazapine, desvenlafaxine, nortriptyline, lorazepam, oxazepam, buspirone, quetiapine, trazodone, promazine, aripiprazole, ziprasidone, olanzapine, risperidone, acrivastine, azelastine, bilastine, bromodiphenhydramine, brompheniramine, buclizine, barbinoxamine, cetirizine, chlorodiphenhydramine, chlorpheniramine, clemastine, cyclizine, cyproheptadine, desloratadine, dexbrompheniramine, dexchlorpheniramine, dimenhydrinate dimetindene, diphenhydramine, doxylamine, ebastine, embramine, fexofenadine, hydroxyzine, levocabastine, levocetirizine, loratadine, meclizine, mirtazapine, olopatadine, orphenadrine, phenindamine, pheniramine, phenyltoloxamine, promethazine, quetiapine, rupatadine, tripelennamine, triprolidine, levocetirizine, desloratadine, pyrilamine, cimetidine, famotidine, lafutidine, nizatidine, ranitidine, roxatidine, tiotidine, clobenpropit, ABT-239, ciproxifan, conessine, A-349,821, thioperamide, thioperamide, JNJ 7777120, VUF-6002, quazepam, chlordiazepoxide, flurazepam, alprazolam, clorazepate, diazepam, estazolam, clonazepam, oxazepam, triazolam, lorazepam, temazepam, clobazam, midazolam, or oxytocin.

In some embodiments, the cyclobenzaprine is the free base or a pharmaceutically acceptable salt of the free base. In some embodiments, the cyclobenzaprine is the free base. In some embodiments, the cyclobenzaprine is a pharmaceutically acceptable salt. In some embodiments, the cyclobenzaprine is an acid salt. In some embodiments, the cyclobenzaprine acid salt is cyclobenzaprine hydrochloride. In some embodiments, the pharmaceutical composition comprises a pharmaceutically acceptable salt of cyclobenzaprine and a basifying agent.

In some embodiments, the composition of this disclosure comprises between 0.1 mg and 30 mg of cyclobenzaprine or a pharmaceutically acceptable salt thereof. In some embodiments, the composition comprises between 1 mg and 20 mg of cyclobenzaprine or a pharmaceutically acceptable salt thereof. In some embodiments, the composition comprises less than 10 mg of cyclobenzaprine or a pharmaceutically acceptable salt thereof. In some embodiments, the composition comprises less than 5 mg of cyclobenzaprine or a pharmaceutically acceptable salt thereof. In some embodiments, the composition comprises about 5.6 mg of cyclobenzaprine or a pharmaceutically acceptable salt thereof. In some embodiments, the composition comprises about 2.8 mg of cyclobenzaprine or a pharmaceutically acceptable salt thereof. In some embodiments, the composition comprises about 5.6 mg of cyclobenzaprine-HCl. In some embodiments, the composition comprises about 2.8 mg of cyclobenzaprine-HCl.

In some embodiments, the pharmaceutical composition of this disclosure is formulated for sublingual, buccal, oral, suppository, intravenous, intramuscular, subcutaneous, inhalational, intranasal, thin film, transdermal, parenteral, rectal, or vaginal administration. In some embodiments, the pharmaceutical composition is formulated for sublingual administration. In some embodiments, the pharmaceutical composition is formulated for buccal administration. In some embodiments, the pharmaceutical composition is formulated for oral administration. In some embodiments, the pharmaceutical composition is formulated for suppository administration. In some embodiments, the pharmaceutical composition is formulated for intravenous administration. In some embodiments, the pharmaceutical composition is formulated for intramuscular administration. In some embodiments, the pharmaceutical composition is formulated for subcutaneous administration. In some embodiments, the pharmaceutical composition is formulated for inhalational administration. In some embodiments, the pharmaceutical composition is formulated for intranasal administration. In some embodiments, the pharmaceutical composition is formulated for transdermal administration. In some embodiments, the pharmaceutical composition is formulated for parenteral administration. In some embodiments, the pharmaceutical composition is formulated for rectal administration. In some embodiments, the pharmaceutical composition is formulated for vaginal administration.

In some embodiments, the dosage form of the compositions of this disclosure is a tablet, a film, a thin film, a liquid, powder, or a spray solution. In some embodiments, the dosage form is a tablet. In some embodiments, the dosage form is a film. In some embodiments, the dosage form is a thin film. In some embodiments, the dosage form is a liquid. In some embodiments, the dosage form is a powder. In some embodiments, the dosage form is a spray solution. In some embodiments, the dosage form is a sublingual tablet, a sublingual film, a sublingual liquid, sublingual powder, or a sublingual spray solution. In some embodiments, the dosage form is a sublingual tablet. In some embodiments, the dosage form is a sublingual film. In some embodiments, the dosage form is a sublingual liquid. In some embodiments, the dosage form is sublingual powder. In some embodiments, the dosage form is a sublingual spray solution.

### EXAMPLES

### Example 1

Effects on Female Sexual Functioning of a Low Dose, Sublingual Formulation of Cyclobenzaprine in Subjects with Military-Related PTSD after 12 Weeks of Treatment

In this 12-week, multicenter, adaptive design, randomized controlled fixed-dose trial, 5.6 mg cyclobenzaprine-HCl (2 x 2.8 mg sublingual tablets, comprising a 75% ± 2% cyclobenzaprine-HCl and 25% ± 2% mannitol eutectic and a potassium phosphate dibasic anhydrous basifying agent) taken daily at bedtime was compared to placebo for the treatment of PTSD at 45 U.S. sites. Eligible participants (males and females) were 18-75 years of age, had experienced DSM-5 PTSD Criterion A-qualifying trauma(s) during military service since 2001, met DSM-5-defined PTSD by the Clinician-Administered PTSD Scale (CAPS-5), had a baseline CAPS-5 severity score ≥ 33, and were free of antidepressants, and free or washed off other psychotropic medications. A pre-planned interim analysis was conducted when about half the originally planned sample of 550 participants had outcome data.

The study was stopped early after the interim analysis indicated there was a low probability of achieving a significant separation between treatment groups on the CAPS-5 primary endpoint. Thus, all analyses were conducted on the interim analysis sample of 274 participants. The modified intention-to-treat (mITT) population, which included all randomized subjects with at least one post-baseline CAPS-5, included 125 participants treated with 5.6 mg dose per day the above cyclobenzaprine-HCl sublingual formulation and 127 participants on placebo were studied. The female subgroup of the study, which included 10 participants treated with the 5.6 mg dose per day the above cyclobenzaprine-HCl sublingual formulation and 17 participants on placebo, demonstrated a large observed mean improvement in CAPS-5 over placebo (by -9.1 units after 12 weeks of treatment).

Sexual functioning was measured by Changes in Sexual Functioning Questionnaire short-form (CSFQ-14) which is a validated 14-item measure (Keller, McGarvey, Clayton, 2006). Parallel gender-specific versions are administered to males (CSFQ-14-M) and females (CSFQ-14-F), and each sex was analyzed separately. In addition to a total sexual functioning score, there are five sub-scales: desire/frequency, desire/interest, arousal/excitement, orgasm/complete, and pleasure. The scales range from (*never*) to (*every day*) and higher scores reflect greater levels of sexual functioning.

Moderate effect sizes were found on the CSFQ-14-F total score and on 4 of 5 subscales between females treated with the 5.6 mg dose (N=8) and placebo (N=16). Due to sample size differences between the active treatment and placebo females groups, the results are better characterized by Hedge's g effect sizes. Specifically, there was a moderate effect on the total score g = 0.49 (95% CI -1.34, 0.37), desire/interest subscale g = 0.63 (95% CI - 1.49, 0.25), pleasure subscale g = 0.54 (95% CI -1.40, 0.33), desire/frequency subscale g = 0.46 (95% CI -1.31, 0.40) and arousal/excitement subscale *g* = 0.55 (95% CI -1.41, 0.32). No effect was found on the orgasm/completion scale. Overall, adverse events (AE) in the study were comparable to prior studies with a similar 5.6 mg cyclobenzaprine-HCl dose. The most frequent AE was oral hypoaesthesia (tongue/mouth numbness), related to the site of administration, which was generally transient (<60 min post administration) and never rated as severe. The most common systemic AE was somnolence, also never rated as severe. Two participants on placebo and 8 participants who received the 5.6 mg cyclobenzaprine-HCl dose, had at least one AE leading to study discontinuation.

The results in the female group suggests that the 5.6 mg daily dose of the cyclobenzaprine-HCl composition has a clinically meaningful effect on improvement of overall sexual functioning, the frequency of sexual acts, interest in sexual experiences, ease of arousal, and current enjoyment of sex over placebo in females with military-related PTSD. This effect was strong for arousal/excitement, which is a primary form of female sexual dysfunction with limited effective and safe treatment options available (Goldstein, 2000).

### Example 2

Effects on Female Sexual Functioning of a Sublingual Formulation of Cyclobenzaprine-HCl in Military and Civilian Phase 3 PTSD Trials: Preliminary Evidence for a Female-Specific Improvement in Sexual Functioning after 12 Weeks

Three randomized, placebo-controlled and double-blind clinical trials of the above described 5.6 mg cyclobenzaprine-HCl sublingual formulation (2 x 2.8 mg tablets) were performed, a Phase 2 study and a Phase 3 study in military-related PTSD, and a Phase 3 study in predominantly civilian PTSD. All three trials showed encouraging activity using the 5.6 mg cyclobenzaprine-HCl dose on clinician- and patient-rated global PTSD symptoms (Clinician Global Impression [CGI] and Patient Global Impression of Change [PGIC]). Given the very low rates of adverse events related to sexual function in both drug and placebo groups in the Phase 2 study, a systematic study was undertaken to assess the effects of the treatment on female sexual functioning in subsequent Phase 3 studies. The present retrospective analysis examined the activity of the cyclobenzaprine-HCl sublingual formulation on the Changes in Sexual Functioning Questionnaire short form (CSFQ-14) in the two Phase 3 studies.

In the Phase 3 study in military-related PTSD, eligible participants (males and females) were 18-75 years of age, had experienced DSM-5 PTSD Criterion A-qualifying trauma(s) during military service since 2001, met DSM-5-defined PTSD by the CAPS-5, had a baseline CAPS-5 severity score ≥ 33, and were free of antidepressants, and free or washed off other psychotropic medications. In the Phase 3 study in predominantly civilian PTSD, the inclusion criteria were broadened to include civilian participants with current PTSD, as determined by the CAPS-5. Accordingly, this study included 94% civilian trauma with a minimum baseline severity score of ≥ 33 on the CAPS-5. Both studies were stopped early after the interim analysis indicated there was a low probability of achieving a significant separation between treatment groups on the CAPS-5 primary endpoint. Thus, analyses were conducted on the interim analysis sample of 252 participants in the military Phase 3 study (89% male [n=225 M; n=27 F]; 100% military PTSD) and 143 subjects in the civilian Phase 3 study (79% female [n=129 F; n=34 M]; 94% civilian PTSD).

The CSFQ-14 is a validated 14-item measure (Keller, McGarvey, Clayton, 2006) that has male and female versions which were analyzed separately. In addition to a total sexual functioning score, there are five sub-scales: desire/frequency, desire/interest, arousal/excitement, orgasm/complete, and pleasure. The items range on 5-point Likert scale from (never) to (every day). Higher scores reflect greater levels of sexual functioning.

In the Phase 3 study of predominantly male and all military-related PTSD, the sample size (N=24) of female completers was small and the study was not powered to detect differences in subgroups. Therefore, effect sizes, as characterized by Hedges' g, are reported for females treated with the 5.6 cyclobenzaprine-HCl dose (N=8) relative to placebo (N=16) on the CSFQ-14 total score and on 4 of 5 subscales: total score g=0.49 (95% CI -1.34, 0.37), desire/interest subscale g=0.63 (95% CI -1.49, 0.25), pleasure subscale g=0.54 (95% CI -1.40, 0.33), desire/frequency subscale g=0.46 (95% CI -1.31, 0.40) and arousal/excitement subscale g=0.55 (95% CI -1.41, 0.32). No effect was found on the orgasm/completion scale.

In the Phase 3 study of predominantly civilian PTSD subjects, the female completers in the 5.6 mg cyclobenzaprine-HCl dose (N=58) group trended towards improvement on the CSFQ-14-F total score versus placebo (N=55), with a moderate effect size (p=0.07, Hedges' g=0.37 [95% CI 0.00, 0.74]). Although the study was not powered to detect differences in subgroups, small to moderate effect sizes were observed for each of the subscales including: desire/interest g=0.23 (95% CI -0.14, 0.60), desire/frequency g=0.21 (95% CI -0.16, 0.57), pleasure g=0.19 (95% CI - 0.17, 0.56), arousal/excitement g=0.33 (95% CI -0.04, 0.70), and orgasm/completion g=0.29 (95% CI -0.08, 0.66).

Adverse events (AE) in both Phase 3 studies were comparable to prior studies with the 5.6 mg cyclobenzaprine-HCl dose. The most frequent AE was oral hypoaesthesia (tongue/mouth numbness), related to the site of administration, which was generally transient (<60 minutes post administration) and never rated as severe. The most common systemic AE was somnolence, also never rated as severe.

Results in the female groups of the two Phase 3 studies suggest a clinically meaningful trend of improvement in overall sexual functioning in females with PTSD. Results from both studies demonstrated a strong effect for female arousal/excitement, which is a primary form of female sexual dysfunction with limited available treatment options. Although the effect size was larger in the military Phase 3 study, there were relatively few females in that study. In the civilian Phase 3 study, the female sample was larger, and proportionately more female participants in that study reported an index trauma related to sexual trauma.

These above examples indicate that the 5.6 mg cyclobenzaprine-HCl dose of a composition of this disclosure has activity in female SD in military-related PTSD and civilian PTSD.

## Claims

1. A pharmaceutical composition comprising a therapeutically effective amount of cyclobenzaprine or a pharmaceutically acceptable salt thereof and a pharmaceutically acceptable carrier for use in treating or preventing sexual dysfunction in a female subject in need or at risk thereof, wherein the sexual dysfunction is associated with one or more symptoms selected from the group consisting of sexual aversion, low sexual desire or interest, fear of sex, difficulty with arousal, inability to become aroused or maintain arousal during sexual activity, persistent or recurrent difficulty in achieving orgasm after sufficient sexual arousal and ongoing stimulation, and pain associated with sexual stimulation or vaginal contact.

2. A combination for use in treating or preventing sexual dysfunction in a female subject in need or at risk thereof, the combination comprising a therapeutically effective amount of a pharmaceutical composition comprising cyclobenzaprine or a pharmaceutically acceptable salt thereof and a pharmaceutically acceptable carrier and a pharmaceutical composition comprising a therapeutic effective amount of one or more therapeutic agents selected from the group consisting of an estrogen receptor modulator, a 5-hydroxytryptamine 1A (5-HT_{1A}) receptor agonist, a 5-hydroxytryptamine 2A (5-HT_{2A}) antagonist, a steroid agent, a phosphodiesterase inhibitor, a melanocortin receptor agonist, an alpha-1-adrenergic receptor antagonist, a beta-adrenergic antagonist, an anticonvulsant or a mood stabilizer, a selective serotonin reuptake inhibitor, a serotonin-norepinephrine reuptake inhibitor, an antidepressant, an anti-anxiety agent, an antipsychotic, an antihistamine, a benzodiazepine, a psychoactive agent, a barbiturate, lithium, an antihypertensive agent, an antilipid agent, a hormonal agent, a gonadotropin-releasing hormone (GnRh) agonist, a contraceptive, an anticholinergic, an amphetamine, a dopaminergic receptor agonist, an anorexic agent, and a narcotic agent.

3. The combination for the use of claim 2, wherein the pharmaceutical composition of cyclobenzaprine or pharmaceutically acceptable salt thereof and the pharmaceutical composition of the one or more therapeutic agents are in the same dosage form or in separate dosage forms packaged together or packaged separately, wherein the compositions are administered simultaneously or sequentially.

4. The combination for the use of claim 2 or 3, wherein the:
(a) the estrogen receptor modulator is ospemifene;
(b) the 5-HT_{1A} receptor agonist or the 5-HT_{2A} receptor agonist is flibanserin;
(c) the dopaminergic receptor agonist is apomorphine;
(d) the steroid agent is tibolone, estrogen, or testosterone;
(e) the phosphodiesterase inhibitor is sildenafil or tadalafil;
(f) the melanocortin receptor agonist is bremelanotide;
(g) the alpha-1- adrenergic receptor antagonist is prazosin, terazosin, doxazosin, silodosin, alfuzosin, or tamsulosin;
(h) the beta adrenergic receptor antagonist is propranolol, bucindolol, carteolol, carvedilol, labetalol, nadolol, oxprenolol, penbutolol, pindolol, sotalol, timolol, acebutolol, atenolol, betaxolol, bisoprolol, celiprolol, metoprolol, nebivolol, esmolol, butaxamine, ICI-118,551, SR 59230A, or nebivolol;
(i) the anticonvulsant or mood stabilizer is carbamazepine, divalproex, dextromethorphan, gabapentin, lamotrigine, oxcarbazepine, pregabalin, tiagabine, topimarate, or valproate
(j) the selective serotonin reuptake inhibitor is citalopram, escitalopram, fluoxetine, fluvoxamine, paroxetine, or sertraline;
(k) the serotonin-norepinephrine reuptake inhibitor is atomoxetine, duloxetine, desvenlafaxine, levomilnacipran, milnacipran, sibutramine, tramadol, or venlafaxine;
(l) the antidepressant is citalopram, fluoxetine, paroxetine, sertraline, escitalopram, trazodone, venlafaxine, bupropion, duloxetine, amitriptyline, venlafaxine, mirtazapine, desvenlafaxine, or nortriptyline;
(m)the anti-anxiety agent is lorazepam, oxazepam, or buspirone;
(n) the antipsychotic agent is quetiapine, trazodone, promazine, aripiprazole, ziprasidone, olanzapine, or risperidone;
(o) the antihistamine is acrivastine, azelastine, bilastine, bromodiphenhydramine, brompheniramine, buclizine, barbinoxamine, cetirizine, chlorodiphenhydramine, chlorpheniramine, clemastine, cyclizine, cyproheptadine, desloratadine, dexbrompheniramine, dexchlorpheniramine, dimenhydrinate dimetindene, diphenhydramine, doxylamine, ebastine, embramine, fexofenadine, hydroxyzine, levocabastine, levocetirizine, loratadine, meclizine, mirtazapine, olopatadine, orphenadrine, phenindamine, pheniramine, phenyltoloxamine, promethazine, quetiapine, rupatadine, tripelennamine, triprolidine, levocetirizine, desloratadine, pyrilamine, cimetidine, famotidine, lafutidine, nizatidine, ranitidine, roxatidine, tiotidine, clobenpropit, ABT-239, ciproxifan, conessine, A-349,821, thioperamide, thioperamide, JNJ 7777120, or VUF-6002;
(p) the benzodiazepine is quazepam, chlordiazepoxide, flurazepam, alprazolam, clorazepate, diazepam, estazolam, clonazepam, oxazepam, triazolam, lorazepam, temazepam, clobazam, or midazolam; or
(q) the hormonal agent is oxytocin, estrogen, or testosterone.

5. The pharmaceutical composition for the use of claim 1 or the combination for the use of any one of claims 2-4, wherein the pharmaceutically acceptable salt of cyclobenzaprine is cyclobenzaprine-HCl.

6. The pharmaceutical composition or combination for the use of any one of claims 1-5, wherein the cyclobenzaprine or the pharmaceutically acceptable salt thereof is in the form of a mannitol eutectic.

7. The pharmaceutical composition or combination for the use of claim 6, wherein the mannitol eutectic is selected for the group consisting of a 75% ± 2% cyclobenzaprine-HCl and 25% ± 2% mannitol eutectic, a 65% ± 2% cyclobenzaprine-HCl and 35% ± 2% δ-mannitol eutectic, a mixture of a 75% ± 2% cyclobenzaprine-HCl and 25% ± 2% β-mannitol and a 65% ± 2% cyclobenzaprine-HCl and 35% ± 2% δ-mannitol eutectic, and a granule comprising an outer layer of a 65% ± 2% cyclobenzaprine-HCl and 35% ± 2% δ-mannitol eutectic and an inner layer of β-mannitol.

8. The pharmaceutical composition or combination for the use of any one of claims 1-7, wherein the composition comprising a pharmaceutically acceptable salt of cyclobenzaprine further comprises a basifying agent selected from the group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate, disodium citrate and trisodium citrate.

9. The pharmaceutical composition for the use of claim 1 or the combination for the use of any one of claims 2-4, wherein the composition comprising the cyclobenzaprine or pharmaceutically acceptable salt thereof comprises:
(a) between 0.1 mg and 30 mg of cyclobenzaprine or a pharmaceutically acceptable salt thereof;
(b) between 1 mg and 20 mg of cyclobenzaprine or a pharmaceutically acceptable salt thereof;
(c) less than 10 mg of cyclobenzaprine or a pharmaceutically acceptable salt thereof;
(d) less than 5 mg of cyclobenzaprine or a pharmaceutically acceptable salt thereof.

10. The pharmaceutical composition or combination for the use of claim 9, wherein the pharmaceutically acceptable salt of the composition comprising the cyclobenzaprine or pharmaceutically salt thereof is cyclobenzaprine-HCl and the composition comprises about 5.6 mg of that salt.

11. The pharmaceutical composition or combination for the use of claim 9, wherein the pharmaceutically acceptable salt of the composition comprising the cyclobenzaprine or pharmaceutically salt thereof is cyclobenzaprine-HCl and the composition comprises about 2.8 mg of that salt.

12. The pharmaceutical composition or combination for the use of claim 10, wherein the pharmaceutically acceptable salt of the composition comprising the cyclobenzaprine or pharmaceutically salt thereof is cyclobenzaprine-HCl and the composition is administered simultaneously or sequentially in two dosage units, and wherein:
(i) the combined amount of the composition in the two dosage units is about 5.6 mg of that salt; or
(ii) each dosage unit comprises about 2.8 mg of that salt.

13. The pharmaceutical composition or combination for the use of any one of claims 1-12, wherein the pharmaceutical composition is administered daily.

14. The pharmaceutical composition or combination for the use of claim 13, wherein the pharmaceutical composition is formulated for sublingual, buccal, oral, suppository, intravenous, intramuscular, subcutaneous, inhalational, intranasal, thin film, transdermal, parenteral, rectal, or vaginal administration.

15. The pharmaceutical composition for the use of claim 1 or the combination for the use of any one of claims 2-4, wherein the female subject has female genital organs by birth, reconstructive surgery or sex reassignment surgery.

16. The pharmaceutical composition or combination for the use of claim 15, wherein the female subject is premenopausal, perimenopausal, or postmenopausal.

17. The pharmaceutical composition for the use of claim 1 or the combination for the use of any one of claims 2-4, wherein the sexual dysfunction is associated with the use of one or more agents selected from a group consisting of an antidepressant, an anxiolytic, an antihypertensive agent, a chemotherapy agent, a hormonal agent, a corticosteroid agent, an antipsychotic, an antihistamine, a benzodiazepine, a psychoactive agent, a barbiturate, lithium, an antihypertensive agent, an antilipid agent, a gonadotropin-releasing hormone (GnRh) agonist, a contraceptive, an anticholinergic agent, an amphetamine, an anorexic agent, and a narcotic agent.

18. The pharmaceutical composition for the use of claim 1 or the combination for the use of any one of claims 2-4, wherein the sexual dysfunction:
(a) is associated with a medical or mental health condition; or
(b) is a desire disorder, an arousal disorder, an orgasm disorder, or a sexual pain disorder.

19. The pharmaceutical composition or combination for the use of claim 18, wherein the medical condition is selected from a group consisting of:
(a) a cardiovascular disease selected from the group consisting of heart disease, hypertension and peripheral vascular disease;
(b) obesity;
(c) a cancer selected from the group consisting of breast cancer, ovarian cancer, cervical cancer, endometrial cancer, gestational trophoblastic disease, uterine sarcoma, vaginal cancer, vulvar cancer, pancreatic cancer, rectal cancer, renal cell cancer, skin cancer, brain cancer, head and neck cancer, lung cancer, thyroid cancer, bladder cancer, esophageal cancer, mesothelioma, glioblastoma, thymic carcinoma, lymphoma, leukemia, myeloma, hematologic malignancy, and colon or gastrointestinal cancer;
(d) a pulmonary condition selected from the group consisting of pneumonia, tuberculosis, emphysema, pulmonary edema, acute respiratory distress syndrome, pneumoconiosis, pulmonary embolism, pulmonary hypertension, pleural effusion, pneumothorax, and mesothelioma;
(e) a kidney condition selected from the group consisting of Chronic Kidney Disease (CKD), diabetes, anorexia nervosa, high blood pressure, high cholesterol, lupus, multiple myeloma and hemolytic uremic syndrome;
(f) a bladder condition selected from the group consisting of urethritis, interstitial cystitis, and urinary tract infection;
(g) a rectal condition;
(h) a bowel condition;
(i) a hepatic condition selected from the group consisting of hepatitis, fatty liver disease, liver cancer, hemochromatosis, and Wilson disease;
(j) a gynecological condition selected from the group consisting of menopause, peritonitis, uterine retrogression, fibroids, endometritis, uterine cysts, cystocele, rectocele, uterine prolapse, hysterectomy, oophorectomy, salpingectomy, and hormone fluctuation;
(k) an autoimmune disorder selected from the group consisting of multiple sclerosis, type I diabetes, rheumatoid arthritis, psoriasis, systemic lupus erythematosus, Addison's disease, Graves disease, Sjögren's syndrome, Myasthenia gravis, pernicious anemia, and celiac disease;
(l) a hormonal condition associated with menopause, perimenopause, pregnancy, or childbirth;
(m) a viral infection caused by human papilloma virus hepatitis C virus, or herpes simplex virus;
(n) bacterial infection caused by Gardnerella vaginalis;
(o) a parasitic infection; and
(p) a prion infection, or
wherein the mental health condition is one or more conditions selected from the group consisting of:
(a) a psychological condition selected from sexual trauma or abuse, emotional trauma or abuse, or physical trauma or abuse;
(b) a mood disorder selected from a depressive disorder, a bipolar disorder, or a substance-induced disorder;
(c) a trauma and stressor related disorder selected from post-traumatic stress disorder (PTSD), acute stress disorder (ASD), adjustment disorder, or reactive attachment disorder;
(d) a neurodegenerative disorder selected from Mild Cognitive Impairment, amnestic Mild Cognitive Impairment, Parkinson's disease, Huntington's disease, Alzheimer's Disease, dementia, Amyotrophic lateral sclerosis, or motor neuron disease; and
(e) an anxiety disorder selected from panic disorder, generalized anxiety disorder (GAD), a specific phobia, or social phobia.

## Patentansprüche

1. Pharmazeutische Zusammensetzung, umfassend eine therapeutisch wirksame Menge Cyclobenzaprin oder eines pharmazeutisch unbedenklichen Salz davon und einen pharmazeutisch unbedenklichen Träger, zur Verwendung bei der Behandlung oder Prävention sexueller Dysfunktion bei einem bedürftigen oder gefährdeten weiblichen Subjekt, wobei die sexuelle Dysfunktion mit einem oder mehreren aus der aus sexueller Abneigung, geringem sexuellem Verlangen oder Interesse, Angst vor Sex, Schwierigkeiten bei der Erregung, der Unfähigkeit, während der sexuellen Aktivität erregt zu werden oder die Erregung aufrechtzuerhalten, anhaltenden oder wiederkehrenden Schwierigkeiten beim Erreichen eines Orgasmus nach ausreichender sexueller Erregung und fortgesetzter Stimulation sowie Schmerzen im Zusammenhang mit sexueller Stimulation oder vaginalem Kontakt bestehenden Gruppe ausgewählten Symptomen assoziiert ist.

2. Kombination zur Behandlung oder Prävention sexueller Dysfunktion bei einem bedürftigen oder gefährdeten weiblichen Subjekt, wobei die Kombination eine therapeutisch wirksame Menge einer Cyclobenzaprin oder ein pharmazeutisch unbedenkliches Salz davon und einen pharmazeutisch unbedenklichen Träger umfassenden pharmazeutischen Zusammensetzung und einer eine therapeutische wirksame Menge eines oder mehrerer therapeutischer Mittel, ausgewählt aus der aus einem Estrogenrezeptormodulator, einem 5-Hydroxytryptamin-1A(5-HT_{1A})-Rezeptoragonisten, einem 5-Hydroxytryptamin-2A-(5-HT_{2A})-Antagonisten, einem Steroid, einem Phosphodiesterasehemmer, einem Melanocortinrezeptoragonist, einem Antagonisten des alpha-1-adrenergen Rezeptors, einem Antagonisten des beta-adrenergen Antagonisten, einem Antikonvulsivum oder einem Stimmungsstabilisator, einem selektiven Serotonin-Wiederaufnahmehemmer, einem Serotonin-Noradrenalin-Wiederaufnahmehemmer, einem Antidepressivum, einem Anxiolytikum, einem Antipsychotikum, einem Antihistaminikum, einem Benzodiazepin, einem psychoaktiven Wirkstoff, einem Barbiturat, Lithium, einem blutdrucksenkenden Mittel, einem Antilipidmittel, einem Hormon, einem Agonisten des Gonadotropin Releasing Hormon (GnRH), einem Kontrazeptivum, einem Anticholinergikum, einem Amphetamin, einem Agonisten des dopaminergen Rezeptors, einem Appetitzügler und einem Narkotikum bestehenden Gruppe, umfassenden pharmazeutischen Zusammensetzung umfasst.

3. Kombination zur Verwendung nach Anspruch 2, wobei die pharmazeutische Zusammensetzung von Cyclobenzaprin oder einem pharmazeutisch unbedenklichen Salz davon und die pharmazeutische Zusammensetzung des einen oder der mehreren Therapeutika in der gleichen Verabreichungsform oder in separaten Verabreichungsformen zusammen oder getrennt verpackt sind, wobei die Zusammensetzungen gleichzeitig oder aufeinanderfolgend verabreicht werden.

4. Kombination zur Verwendung von Anspruch 2 oder 3, wobei:
(a) der Estrogenrezeptormodulator Ospemifen ist,
(b) der 5-HT_{1A}-Rezeptoragonist oder der 5-HT_{2A}-Rezeptoragonist Flibanserin ist,
(c) der Agonist des dopaminergen Rezeptors Apomorphin ist,
(d) das Steroid Tibolon, Estrogen oder Testosteron ist,
(e) der Phosphodiesterasehemmer Sildenafil oder Tadalafil ist,
(f) der Melanocortinrezeptoragonist Bremelanotid ist,
(g) der Antagonist des alpha-1-Adrenorezeptors Prazosin, Terazosin, Doxazosin, Silodosin, Alfuzosin oder Tamsulosin ist,
(h) der Antagonist des beta-adrenergen Rezeptors Propranolol, Bucindolol, Carteolol, Carvedilol, Labetalol, Nadolol, Oxprenolol, Penbutolol, Pindolol, Sotalol, Timolol, Acebutolol, Atenolol, Betaxolol, Bisoprolol, Celiprolol, Metoprolol, Nepvolol, Esmolol, Butaxamin, ICI-118,551, SR 59230A oder Nebivolol ist,
(i) das Antikonvulsivum oder der Stimmungsstabilisator Carbamazepin, Divalproex, Dextromethorphan, Gabapentin, Lamotrigin, Oxcarbazepin, Pregabalin, Tiagabin, Topiramat oder Valproat ist,
(j) der selektive Serotonin-Wiederaufnahmehemmer Citalopram, Escitalopram, Fluoxetin, Fluvoxamin, Paroxetin oder Sertralin ist,
(k) der Serotonin-Noradrenalin-Wiederaufnahmehemmer Atomoxetin, Duloxetin, Desvenlafaxin, Levomilnacipran, Milnacipran, Sibutramin, Tramadol oder Venlafaxin ist,
(l) das Antidepressivum Citalopram, Fluoxetin, Paroxetin, Sertralin, Escitalopram, Trazodon, Venlafaxin, Bupropion, Duloxetin, Amitriptylin, Venlafaxin, Mirtazapin, Desvenlafaxin oder Nortriptylin ist,
(m) das Anxiolytikum Lorazepam, Oxazepam oder Buspiron ist,
(n) das Antipsychotikum ist Quetiapin, Trazodon, Promazin, Aripiprazol, Ziprasidon, Olanzapin oder Risperidon ist,
(o) das Antihistaminikum Acrivastin, Azelastin, Bilastin, Bromodiphenhydramin, Brompheniramin, Buclizin, Barbinoxamin, Cetirizin, Chlorodiphenhydramin, Chlorpheniramin, Clemastin, Cyclizin, Cyproheptadin, Desloratadin, Dexbrompheniramin, Dexchlorpheniramin, Dimenhydrinat, Dimetinden, Diphenhydramin, Doxylamin, Ebastin, Embramin, Fexofenadin, Hydroxyzin, Levocabastin, Levocetirizin, Loratadin, Meclizin, Mirtazapin, Olopatadin, Orphenadrin, Phenindamin, Pheniramin, Phenyltoloxamin, Promethazin, Quetiapin, Rupatadin, Tripelennamin, Triprolidin, Levocetirizin, Desloratadin, Pyrilamin, Cimetidin, Famotidin, Lafutidin, Nizatidin, Ranitidin, Roxatidin, Tiotidin, Clobenpropit, ABT-239, Ciproxifan, Conessin, A-349,821, Thioperamid, JNJ 7777120 oder VUF-6002 ist,
(p) das Benzodiazepin Quazepam, Chlordiazepoxid, Flurazepam, Alprazolam, Clorazepat, Diazepam, Estazolam, Clonazepam, Oxazepam, Triazolam, Lorazepam, Temazepam, Clobazam oder Midazolam ist oder
(q) das Hormon Oxytocin, Estrogen oder Testosteron ist.

5. Pharmazeutische Zusammensetzung zur Verwendung nach Anspruch 1 oder Kombination zur Verwendung nach einem der Ansprüche 2-4, wobei das pharmazeutisch unbedenkliche Salz von Cyclobenzaprin Cyclobenzaprin-HCl ist.

6. Pharmazeutische Zusammensetzung oder Kombination zur Verwendung nach einem der Ansprüche 1-5, wobei das Cyclobenzaprin oder dessen pharmazeutisch unbedenkliches Salz in Form eines Mannitol-Eutektikums vorliegt.

7. Pharmazeutische Zusammensetzung oder Kombination zur Verwendung von Anspruch 6, wobei das Mannitol-Eutektikum aus der aus einem Eutektikum aus 75 % ± 2 % Cyclobenzaprin-HCl und 25 % ± 2 % Mannitol, einem Eutektikum aus 65 % ± 2 % Cyclobenzaprin-HCl und 35 % ± 2 % 5-Mannitol-Eutektikum, einer Mischung aus 75 % ± 2 % Cyclobenzaprin-HCl und 25 % ± 2 % β-Mannitol und einem Eutektikum aus 65 % ± 2 % Cyclobenzaprin-HCl und 35 % ± 2 % δ-Mannitol sowie einem Granulat, das eine äußere Schicht aus einem Eutektikum aus 65 % ± 2 % Cyclobenzaprin-HCl und 35 % ± 2 % δ-Mannitol und eine innere Schicht aus β-Mannitol umfasst, bestehenden Gruppe ausgewählt ist.

8. Pharmazeutische Zusammensetzung oder Kombination zur Verwendung nach einem der Ansprüche 1-7, wobei die ein pharmazeutisch unbedenkliches Salz von Cyclobenzaprin umfassende Zusammensetzung weiterhin ein aus der aus Kaliumdihydrogenphosphat, Dikaliumhydrogenphosphat, Kaliumphosphat, Natriumcarbonat, Natriumhydrogencarbonat, Calciumcarbonat, Calciumhydrogencarbonat, TRIS-Puffer, Natriumdihydrogenphosphat, Dinatriumhydrogenphosphat, Natriumhydrogenphosphat, Kaliumcarbonat, Kaliumhydrogencarbonat, Kaliumacetat, Natriumacetat, Dikaliumcitrat, Kaliumcitrat, Dinatriumcitrat und Natriumcitrat bestehenden Gruppe ausgewähltes Basifizierungsmittel umfasst.

9. Pharmazeutische Zusammensetzung zur Verwendung nach Anspruch 1 oder die Kombination zur Verwendung eines der Ansprüche 2-4, wobei die das Cyclobenzaprin oder das pharmazeutisch unbedenkliche Salz davon umfassende Zusammensetzung Folgendes umfasst:
(a) 0,1 mg bis 30 mg Cyclobenzaprin oder ein pharmazeutisch unbedenkliches Salz davon,
(b) 1 mg bis 20 mg Cyclobenzaprin oder ein pharmazeutisch unbedenkliches Salz davon,
(c) weniger als 10 mg Cyclobenzaprin oder ein pharmazeutisch unbedenkliches Salz davon,
(d) weniger als 5 mg Cyclobenzaprin oder ein pharmazeutisch unbedenkliches Salz davon.

10. Pharmazeutische Zusammensetzung oder Kombination zur Verwendung nach Anspruch 9, wobei das pharmazeutisch unbedenkliche Salz der das Cyclobenzaprin oder das pharmazeutisch unbedenkliche Salz davon umfassenden Zusammensetzung Cyclobenzaprin-HCl ist und die Zusammensetzung etwa 5,6 mg dieses Salzes umfasst.

11. Pharmazeutische Zusammensetzung oder Kombination zur Verwendung nach Anspruch 9, wobei das pharmazeutisch unbedenkliche Salz der das Cyclobenzaprin oder das pharmazeutisch unbedenkliche Salz davon umfassenden Zusammensetzung Cyclobenzaprin-HCl ist und die Zusammensetzung etwa 2,8 mg dieses Salzes umfasst.

12. Pharmazeutische Zusammensetzung oder Kombination zur Verwendung nach Anspruch 10, wobei das pharmazeutisch unbedenkliche Salz der das Cyclobenzaprin oder das pharmazeutisch unbedenkliche Salz davon umfassenden Zusammensetzung Cyclobenzaprin-HCl ist und die Zusammensetzung gleichzeitig oder aufeinanderfolgend in zwei Dosiseinheiten verabreicht wird, und wobei:
(i) die kombinierte Menge der Zusammensetzung in den beiden Dosiseinheiten etwa 5,6 mg dieses Salzes beträgt oder
(ii) jede Dosiereinheit etwa 2,8 mg dieses Salzes umfasst.

13. Pharmazeutische Zusammensetzung oder Kombination zur Verwendung nach einem der Ansprüche 1-12, wobei die pharmazeutische Zusammensetzung täglich verabreicht wird.

14. Pharmazeutische Zusammensetzung oder Kombination zur Verwendung nach Anspruch 13, wobei die pharmazeutische Zusammensetzung für die sublinguale, bukkale, orale, Zäpfchen-, intravenöse, intramuskuläre, subkutane, inhalative, intranasale, Dünnfilm-, transdermale, parenterale, rektale oder vaginale Verabreichung formuliert ist.

15. Pharmazeutische Zusammensetzung zur Verwendung nach Anspruch 1 oder Kombination zur Verwendung nach einem der Ansprüche 2-4, wobei das weibliche Subjekt weibliche Genitalorgane durch Geburt, Rekonstruktionschirurgie oder Geschlechtsumwandlungschirurgie aufweist.

16. Pharmazeutische Zusammensetzung oder Kombination zur Verwendung nach Anspruch 15, wobei das weibliche Subjekt prämenopausal, perimenopausal oder postmenopausal ist.

17. Pharmazeutische Zusammensetzung zur Verwendung nach Anspruch 1 oder Kombination zur Verwendung nach einem der Ansprüche 2-4, wobei die sexuelle Dysfunktion mit der Verwendung eines oder mehrerer aus der aus einem Antidepressivum, einem Anxiolytikum, einem Antihypertensivum, einem Chemotherapeutikum, einem hormonellen Mittel, einem Kortikosteroid, einem Antipsychotikum, einem Antihistaminikum, einem Benzodiazepin, einem psychoaktiven Mittel, einem Barbiturat, Lithium, einem blutdrucksenkenden Mittel, einem Antilipidmittel, einem Agonisten des Gonadotropin Releasing Hormon (GnRH), einem Kontrazeptivum, einem Anticholinergikum, einem Amphetamin, einem Appetitzügler und einem Betäubungsmittel bestehenden Gruppe ausgewählten Mittel assoziiert ist.

18. Pharmazeutische Zusammensetzung zur Verwendung nach Anspruch 1 oder Kombination zur Verwendung nach einem der Ansprüche 2-4, wobei die sexuelle Dysfunktion:
(a) mit einem medizinischen oder psychischen Leiden assoziiert ist oder
(b) eine Sehstörung, eine Erregungsstörung, eine Orgasmusstörung oder eine sexuelle Schmerzstörung ist.

19. Pharmazeutische Zusammensetzung oder Kombination zur Verwendung nach Anspruch 18, wobei das medizinische Leiden ausgewählt ist aus einer Gruppe bestehend aus:
(a) Herz-Kreislauf-Erkrankungen, ausgewählt aus der Gruppe bestehend aus Herzerkrankungen, Hypertonie und peripherer Gefäßerkrankung,
(b) Adipositas,
(c) einer Krebserkrankung, ausgewählt aus der Gruppe bestehend aus Brustkrebs, Eierstockkrebs, Gebärmutterhalskrebs, Endometriumkarzinom, gestationeller Trophoblastenerkrankung, Uterussarkom, Vaginalkrebs, Vulvakrebs, Bauchspeicheldrüsenkrebs, Rektumkrebs, Nierenzellkrebs, Hautkrebs, Hirntumor, Kopf-Hals-Krebs, Lungenkrebs, Schilddrüsenkrebs, Blasenkrebs, Speiseröhrenkrebs, Mesotheliom, Glioblastom, Thymuskarzinom, Lymphom, Leukämie, Myelom, hämatologischen Malignitäten sowie Kolon- oder Magen-Darm-Krebs,
(d) einem Lungenleiden, ausgewählt aus der Gruppe bestehend aus Lungenentzündung, Tuberkulose, Emphysem, Lungenödem, akutem Atemnotsyndrom, Pneumokoniose, Lungenembolie, pulmonale Hypertonie, Pleuraerguss, Pneumothorax und Mesotheliom,
(e) einem Nierenleiden, ausgewählt aus der Gruppe bestehend aus chronischen Nierenerkrankung (Chronic Kidney Disease, CKD), Diabetes, Anorexia nervosa, Bluthochdruck, hohem Cholesterinspiegel, Lupus, multiplem Myelom und hämolytisch-urämischem Syndrom,
(f) eine Blasenerkrankung, ausgewählt aus der Gruppe bestehend aus Urethritis, interstitieller Zystitis und Harnwegsinfektion,
(g) einem Rektalleiden,
(h) einer Darmerkrankung,
(i) einer Lebererkrankung, ausgewählt aus der Gruppe bestehend aus Hepatitis, Fettleber, Leberkrebs, Hämochromatose und Wilson-Krankheit,
(j) einer gynäkologischen Erkrankung, ausgewählt aus der Gruppe bestehend aus Menopause, Peritonitis, Uterusretrogression, Myomen, Endometritis, Uteruszysten, Zystozele, Rektozele, Uterusprolaps, Hysterektomie, Oophorektomie, Salpingektomie und Hormonfluktuation,
(k) einer Autoimmunerkrankung, ausgewählt aus der Gruppe bestehend aus multipler Sklerose, Typ-I-Diabetes, rheumatoider Arthritis, Psoriasis, systemischem Lupus erythematodes, Morbus Addison, Morbus Basedow, Sjögren-Syndrom, Myasthenia gravis, perniziöser Anämie und Zöliakie,
(l) einem hormonellen Leiden, das mit den Wechseljahren, Perimenopause, Schwangerschaft oder Geburt assoziiert ist,
(m) einer Virusinfektion, die durch das humane Papillomavirus Hepatitis C oder Herpes simplex Virus verursacht wird,
(n) einer durch Gardnerella vaginalis verursachten bakteriellen Infektion,
(o) einer parasitären Infektion und
(p) einer Prioneninfektion, oder
wobei es sich bei dem psychischen Leiden um ein oder mehrere Leiden handelt, die aus der aus den folgenden bestehenden Gruppe ausgewählt sind:
(a) einer psychischen Erkrankung, ausgewählt aus sexuellem Trauma oder sexuellem Missbrauch, emotionalem Trauma oder emotionalem Missbrauch oder körperlichem Trauma oder körperlichem Missbrauch,
(b) einer Stimmungsstörung, ausgewählt aus einer depressiven Störung, einer bipolaren Störung oder einer substanzinduzierten Störung,
(c) einer Trauma- und Stressstörung, ausgewählt aus posttraumatischen Belastungsstörungen (Post-Traumatic Stress Disorder, PTSD), akuten Belastungsstörungen (Acute Stress Disorder, ASD), Anpassungsstörungen oder reaktiven Bindungsstörungen,
(d) einer neurodegenerative Erkrankung, ausgewählt aus leichter kognitiver Störung, amnestischer leichter kognitiver Störung, Parkinson-Krankheit, Huntington-Krankheit, Alzheimer-Krankheit, Demenz, amyotropher Lateralsklerose oder Motoneuronkrankheit, und
(e) einer Angststörung, ausgewählt aus Panikstörung, generalisierter Angststörung (Generalized Anxiety Disorder, GAD), einer spezifischen Phobie oder sozialer Phobie.

## Revendications

1. Composition pharmaceutique comprenant une quantité thérapeutiquement efficace de cyclobenzaprine ou d'un sel pharmaceutiquement acceptable de celle-ci et un excipient pharmaceutiquement acceptable pour une utilisation dans le traitement ou la prévention d'un dysfonctionnement sexuel chez une femme qui en a besoin ou est à risque d'un dysfonctionnement sexuel, dans laquelle le dysfonctionnement sexuel est associé à un ou plusieurs symptômes choisis dans le groupe constitué par l'aversion sexuelle, le faible désir ou intérêt sexuel, la peur du sexe, les difficultés d'excitation, l'incapacité à s'exciter ou à maintenir l'excitation pendant l'activité sexuelle, les difficultés persistantes ou récurrentes à atteindre l'orgasme après une excitation sexuelle suffisante et une stimulation continue, et la douleur associée à la stimulation sexuelle ou au contact vaginal.

2. Combinaison destinée à être utilisée dans le traitement ou la prévention d'un dysfonctionnement sexuel chez une femme qui en a besoin ou est à risque d'un dysfonctionnement sexuel, la combinaison comprenant une quantité thérapeutiquement efficace d'une composition pharmaceutique comprenant de la cyclobenzaprine ou un sel pharmaceutiquement acceptable de celle-ci et un support pharmaceutiquement acceptable et une composition pharmaceutique comprenant une quantité thérapeutiquement efficace d'un ou plusieurs agents thérapeutiques choisis dans le groupe constitué par un modulateur des récepteurs d'œstrogènes, un agoniste des récepteurs 5-hydroxytryptamine 1A (5-HT_{1A}), un antagoniste des récepteurs 5-hydroxytryptamine 2A (5-HT_{2A}), un agent stéroïdien, un inhibiteur de la phosphodiestérase, un agoniste des récepteurs de la mélanocortine, un antagoniste des récepteurs α1-adrénergiques, un antagoniste des récepteurs β-adrénergiques, un anticonvulsivant ou un thymorégulateur, un inhibiteur sélectif de la recapture de la sérotonine, un inhibiteur de la recapture de la sérotonine-noradrénaline, un antidépresseur, un anxiolytique, un antipsychotique, un antihistaminique, une benzodiazépine, un psychotrope, un barbiturique, du lithium, un antihypertenseur, un hypolipémiant, un agent hormonal, un agoniste de la GnRH, un contraceptif, un anticholinergique, une amphétamine, un récepteur dopaminergique agoniste, un agent anorexigène et un agent narcotique.

3. Combinaison pour l'utilisation selon la revendication 2, dans laquelle la composition pharmaceutique de cyclobenzaprine ou d'un sel pharmaceutiquement acceptable de celle-ci et la composition pharmaceutique du ou des agents thérapeutiques sont sous la même forme posologique ou sous des formes posologiques distinctes emballées ensemble ou emballées séparément, dans laquelle les compositions sont administrées simultanément ou séquentiellement.

4. Combinaison pour l'utilisation selon la revendication 2 ou 3, dans laquelle :
(a) le modulateur des récepteurs d'œstrogènes est ospémifène;
(b) l'agoniste des récepteurs 5-HT_{1A} ou l'agoniste des récepteurs 5-HT_{2A} est flibansérine;
(c) l'agoniste des récepteurs dopaminergiques est apomorphine;
(d) l'agent stéroïdien est tibolone, œstrogène, ou testostérone;
(e) l'inhibiteur de phosphodiestérase est le sildénafil ou le tadalafil;
(f) l'agoniste des récepteurs de la mélanocortine est le bremélanotide;
(g) l'antagoniste des récepteurs alpha-1-adrénergiques est la prazosine, la térazosine, la doxazosine, la silodosine, l'alfuzosine, ou la tamsulosine;
(h) l'antagoniste des récepteurs bêta-adrénergiques est propranolol, bucindolol, cartéolol, carvédilol, labétalol, nadolol, oxprénolol, penbutolol, pindolol, sotalol, timolol, acébutolol, aténolol, bétaxolol, bisoprolol, céliprolol, métoprolol, nébivolol, esmolol, butaxamine, ICI-118,551, SR 59230A ou nébivolol ;
(i) l'anticonvulsivant ou le stabilisant de l'humeur est carbamazépine, divalproex, dextrométhorphane, gabapentine, lamotrigine, oxcarbazépine, prégabaline, tiagabine, topimarate ou valproate
(j) l'inhibiteur sélectif de recapture de la sérotonine est citalopram, escitalopram, fluoxétine, fluvoxamine, paroxétine ou sertraline ;
(k) l'inhibiteur de recapture de la sérotonine-norépinéphrine est l'atomoxétine, la duloxétine, la desvenlafaxine, le lévomilnacipran, le milnacipran, la sibutramine, le tramadol ou la venlafaxine ;
(l) l'antidépresseur est citalopram, fluoxétine, paroxétine, sertraline, escitalopram, trazodone, venlafaxine, bupropion, duloxétine, amitriptyline, venlafaxine, mirtazapine, desvenlafaxine ou nortriptyline ;
(m) l'agent anxiolytique est le lorazépam, l'oxazépam ou la buspirone;
(n) l'agent antipsychotique est quétiapine, trazodone, promazine, aripiprazole, ziprasidone, olanzapine ou rispéridone ;
(o) l'antihistaminique est acrivastine, azélastine, bilastine, bromodiphénhydramine, bromphéniramine, buclizine, barbinoxamine, cétirizine, chlorodiphénhydramine, chlorphéniramine, clémastine, cyclizine, cyproheptadine, desloratadine, dexbromphéniramine, dexchlorphéniramine, diménhydrinate de dimétindène, diphénhydramine, doxylamine, ébastine, embramine, fexofénadine, hydroxyzine, lévocabastine, lévocétirizine, loratadine, méclizine, mirtazapine, olopatadine, orphénadrine, phénindamine, phéniramine, phényltoloxamine, prométhazine, quétiapine, rupatadine, tripélennamine, triprolidine, lévocétirizine, desloratadine, pyrilamine, cimétidine, famotidine, lafutidine, nizatidine, ranitidine, roxatidine, tiotidine, clobenpropit, ABT-239, ciproxifan, conessine, A-349,821, thiopéramide, thiopéramide, JNJ 7777120 ou VUF-6002 ;
(p) la benzodiazépine est quazépam, chlordiazépoxide, flurazépam, alprazolam, clorazépate, diazépam, estazolam, clonazépam, oxazépam, triazolam, lorazépam, témazépam, clobazam ou midazolam ; ou
(q) l'agent hormonal est l'ocytocine, l'œstrogène ou la testostérone.

5. Composition pharmaceutique pour l'utilisation selon la revendication 1 ou combinaison pour l'utilisation selon l'une quelconque des revendications 2 à 4, dans laquelle le sel pharmaceutiquement acceptable de cyclobenzaprine est cyclobenzaprine-HCl.

6. Composition pharmaceutique ou combinaison pour l'utilisation selon l'une quelconque des revendications 1 à 5, dans laquelle la cyclobenzaprine ou le sel pharmaceutiquement acceptable de celle-ci est sous la forme d'un eutectique de mannitol.

7. Composition pharmaceutique ou combinaison pour l'utilisation selon la revendication 6, dans laquelle l'eutectique de mannitol est choisi dans le groupe constitué par un 75 % ± 2 % cyclobenzaprine-HCl et 25 % ± 2 % eutectique de mannitol, un 65 % ± 2 % cyclobenzaprine-HCl et 35 % ± 2 % δ-eutectique de mannitol, un mélange d'un 75 % ± 2 % cyclobenzaprine-HCl et 25 % ± 2 % β-mannitol et d'un 65 % ± 2 % cyclobenzaprine-HCl et 35 % ± 2 % δ-eutectique de mannitol, et un granule comprenant une couche extérieure d'un 65 % ± 2 % cyclobenzaprine-HCl et 35 % ± 2 % δ-eutectique de mannitol et une couche intérieure de β-mannitol.

8. Composition pharmaceutique ou combinaison pour l'utilisation selon l'une quelconque des revendications 1 à 7, dans laquelle la composition comprenant un sel pharmaceutiquement acceptable de cyclobenzaprine comprend en outre un agent alcalinisant choisi dans le groupe constitué par phosphate monopotassique, phosphate dipotassique, phosphate tripotassique, carbonate de sodium, bicarbonate de sodium, carbonate de calcium, bicarbonate de calcium, tampon TRIS, phosphate monosodique, phosphate disodique, phosphate trisodique, carbonate de potassium, bicarbonate de potassium, acétate de potassium, acétate de sodium, citrate dipotassique, citrate tripotassique, citrate disodique et citrate trisodique.

9. Composition pharmaceutique pour l'utilisation selon la revendication 1 ou combinaison pour l'utilisation selon l'une quelconque des revendications 2 à 4, dans laquelle la composition comprenant la cyclobenzaprine ou un sel pharmaceutiquement acceptable de celle-ci comprend :
(a) entre 0,1 mg et 30 mg de cyclobenzaprine ou d'un sel pharmaceutiquement acceptable de celle-ci;
(b) entre 1 mg et 20 mg de cyclobenzaprine ou d'un sel pharmaceutiquement acceptable de celle-ci;
(c) moins de 10 mg de cyclobenzaprine ou d'un sel pharmaceutiquement acceptable de celle-ci;
(d) moins de 5 mg de cyclobenzaprine ou d'un sel pharmaceutiquement acceptable de celle-ci.

10. Composition pharmaceutique ou combinaison pour l'utilisation selon la revendication 9, dans laquelle le sel pharmaceutiquement acceptable de la composition comprenant la cyclobenzaprine ou un sel pharmaceutiquement acceptable de celle-ci est la cyclobenzaprine-HCl et la composition comprend environ 5,6 mg de ce sel.

11. Composition pharmaceutique ou combinaison pour l'utilisation selon la revendication 9, dans laquelle le sel pharmaceutiquement acceptable de la composition comprenant la cyclobenzaprine ou un sel pharmaceutiquement acceptable de celle-ci est la cyclobenzaprine-HCl et la composition comprend environ 2,8 mg de ce sel.

12. Composition pharmaceutique ou combinaison pour l'utilisation selon la revendication 10, dans laquelle le sel pharmaceutiquement acceptable de la composition comprenant la cyclobenzaprine ou son sel pharmaceutiquement acceptable est la cyclobenzaprine-HCl et la composition est administrée simultanément ou séquentiellement en deux unités posologiques, et dans laquelle :
(i) la quantité combinée de la composition dans les deux unités posologiques est d'environ 5,6 mg de ce sel ; ou
(ii) chaque unité posologique comprend environ 2,8 mg de ce sel.

13. Composition pharmaceutique ou combinaison pour l'utilisation selon l'une quelconque des revendications 1 à 12, dans laquelle la composition pharmaceutique est administrée quotidiennement.

14. Composition pharmaceutique ou combinaison pour l'utilisation selon la revendication 13, dans laquelle la composition pharmaceutique est formulée pour une administration sublinguale, buccale, orale, par suppositoire, intraveineuse, intramusculaire, sous-cutanée, par inhalation, intranasale, en film mince, transdermique, parentérale, rectale ou vaginale.

15. Composition pharmaceutique pour l'utilisation selon la revendication 1 ou combinaison pour l'utilisation selon l'une quelconque des revendications 2 à 4, dans laquelle le sujet féminin a des organes génitaux féminins par naissance, chirurgie reconstructive ou chirurgie de réassignation sexuelle.

16. Composition pharmaceutique ou combinaison pour l'utilisation selon la revendication 15, dans laquelle le sujet féminin est préménopausé, périménopausé ou postménopausé.

17. Composition pharmaceutique pour l'utilisation selon la revendication 1 ou combinaison pour l'utilisation selon l'une quelconque des revendications 2 à 4, dans laquelle le dysfonctionnement sexuel est associé à l'utilisation d'un ou plusieurs agents choisis dans un groupe constitué par un antidépresseur, un anxiolytique, un antihypertenseur, un agent de chimiothérapie, un agent hormonal, un corticostéroïde, un antipsychotique, un antihistaminique, une benzodiazépine, un agent psychoactif, un barbiturique, du lithium, un antihypertenseur, un hypolipémiant, un agoniste de l'hormone de libération des gonadotrophines (GnRH), un contraceptif, un anticholinergique, une amphétamine, un agent anorexique et un agent narcotique.

18. Composition pharmaceutique pour l'utilisation selon la revendication 1 ou combinaison pour l'utilisation selon l'une quelconque des revendications 2 à 4, dans laquelle le dysfonctionnement sexuel :
(a) est associé à un état de santé mentale ou médicale ; ou
(b) est un trouble du désir, un trouble de l'excitation, un trouble de l'orgasme ou un trouble de douleur sexuelle.

19. Composition pharmaceutique ou combinaison pour l'utilisation selon la revendication 18, dans laquelle l'affection médicale est choisie dans un groupe constitué par :
(a) une maladie cardiovasculaire choisie parmi le groupe constitué par une maladie cardiaque, l'hypertension et une maladie vasculaire périphérique ;
(b) l'obésité ;
(c) un cancer choisi dans le groupe constitué par un cancer du sein, un cancer de l'ovaire, un cancer du col de l'utérus, un cancer de l'endomètre, une maladie trophoblastique gestationnelle, un sarcome utérin, un cancer du vagin, un cancer de la vulve, un cancer du pancréas, un cancer du rectum, un cancer des cellules rénales, un cancer de la peau, un cancer du cerveau, un cancer de la tête et du cou, un cancer du poumon, un cancer de la thyroïde, un cancer de la vessie, un cancer de l'œsophage, un mésothéliome, un glioblastome, un carcinome thymique, un lymphome, une leucémie, un myélome, une hémopathie maligne et un cancer du côlon ou gastro-intestinal ;
(d) une affection pulmonaire choisie dans le groupe constitué par une pneumonie, la tuberculose, l'emphysème, l'œdème pulmonaire, le syndrome de détresse respiratoire aiguë, la pneumoconiose, l'embolie pulmonaire, l'hypertension pulmonaire, l'épanchement pleural, le pneumothorax et le mésothéliome ;
(e) une affection rénale choisie parmi le groupe constitué de la maladie rénale chronique (MRC), du diabète, de l'anorexie mentale, de l'hypertension artérielle, de l'hypercholestérolémie, du lupus, du myélome multiple et du syndrome hémolytique et urémique ;
(f) une affection de la vessie choisie parmi le groupe constitué de l'urétrite, de la cystite interstitielle et de l'infection des voies urinaires ;
(g) une affection rectale ;
(h) une affection intestinale;
(i) une affection hépatique choisie parmi le groupe constitué de l'hépatite, de la stéatose hépatique, du cancer du foie, de l'hémochromatose et de la maladie de Wilson ;
(j) une affection gynécologique choisie parmi le groupe constitué de la ménopause, de la péritonite, de la rétrogression utérine, des fibromes, de l'endométrite, des kystes utérins, de la cystocèle, de la rectocèle, du prolapsus utérin, de l'hystérectomie, de l'ovariectomie, de la salpingectomie et des fluctuations hormonales ;
(k) un trouble auto-immun choisi parmi le groupe constitué de la sclérose en plaques, du diabète de type I, de la polyarthrite rhumatoïde, du psoriasis, du lupus érythémateux systémique, de la maladie d'Addison, de la maladie de Graves, du syndrome de Sjögren, de la myasthénie grave, de l'anémie pernicieuse et de la maladie cœliaque ;
(l) une affection hormonale associée à la ménopause, à la périménopause, à la grossesse ou à l'accouchement ;
(m) une infection virale causée par le virus du papillome humain (VPH), le virus de l'hépatite C ou le virus de l'herpès simplex ;
(n) une infection bactérienne causée par Gardnerella vaginalis ;
(o) une infection parasitaire ; et
(p) une infection à prions, ou
dans laquelle l'état de santé mentale est une ou plusieurs affections choisies dans le groupe constitué par :
(a) un trouble psychologique choisi parmi un traumatisme ou des abus sexuels, émotionnels ou physiques ;
(b) un trouble de l'humeur choisi parmi un trouble dépressif, un trouble bipolaire ou un trouble induit par une substance ;
(c) un trouble lié à un traumatisme ou à un facteur de stress choisi parmi le trouble de stress post-traumatique (TSPT), le trouble de stress aigu (TSA), le trouble de l'adaptation ou le trouble de l'attachement réactif ;
(d) un trouble neurodégénératif choisi parmi les troubles cognitifs légers, les troubles cognitifs légers amnésiques, la maladie de Parkinson, la maladie de Huntington, la maladie d'Alzheimer, la démence, la sclérose latérale amyotrophique ou la maladie du motoneurone ; et
(e) un trouble anxieux choisi parmi le trouble panique, le trouble d'anxiété généralisée (TAG), une phobie spécifique ou la phobie sociale.
